# EUROPEAN PATENT APPLICATION

(11) **EP 4 717 278 A1**
(43) Date of publication of application: **01.04.2026**
(21) Application number: 24306602.4
(22) Date of filing: 30.09.2024
(51) Int. Cl.: A61K 49/10, A61K 49/18, A61K 41/00, C07F 17/02

(54) **NANOVECTORS FOR MAGNETIC RESONANCE IMAGING**

(71) Applicant: Commissariat à l'Energie Atomique et aux Energies Alternatives, 75015 Paris (FR); Université Paris-Saclay, 91190 Gif-sur-Yvette (FR); Centre National de la Recherche Scientifique, 75016 Paris (FR); Institut National de la Recherche pour l'Agriculture, l'Alimentation et l'Environnement, 75007 Paris (FR)
(72) Inventor: DORIS, Eric, 91191 Gif-Sur-Yvette (FR); GRAVEL, Edmond, 91191 Gif-Sur-Yvette (FR); LETERRIER, Claire, 91191 Gif-Sur-Yvette (FR); MERIAUX, Sébastien, 91191 Gif-Sur-Yvette (FR)
(74) Representative: Ipsilon

(57) **Abstract**

The invention relates to the field of magnetic resonance imaging.

The invention relates to a nanovector comprising:
- an external layer comprising a plurality of amphiphilic molecules, said amphiphilic molecules comprising at least one hydrophobic moiety linked to at least one hydrophilic moiety, and
- a core comprising at least one organometallic complex having a paramagnetic property that can be activated by oxidation, said organometallic complex being functionalized by at least one hydrophobic moiety,
the oxidation of the organometallic complex being controllable by light.

The invention also relates to a method of magnetic resonance imaging.

## Description

### TECHNICAL FIELD

The invention relates to the field of magnetic resonance imaging, also called MRI, in particular ¹H-MRI, ¹⁹F-MRI, ¹³C-MRI, ²H-MRI or ³¹P-MRI. In particular, the invention relates to the field of diagnostics.

### BACKGROUND OF THE INVENTION

MRI is a key technique to obtain resolved images of deep soft tissues. MRI is non-invasive and does not rely on the use of radioactive molecules or ionizing radiations, as opposed to other clinical imaging procedures, such as positon emission tomography or X-ray computed tomography. Conventional MRI (*i.e.* proton MRI) provides contrasted images that simply reflect the relaxation rates of water protons in different tissues. Contrast agents are used to enhance the contrast between healthy and pathological tissues and thus provide exploitable imaging data.

Wahsner et al. (Chem. Rev., 2019, 119, 957) describes the chemistry of MRI contrast agents such as paramagnetic (e.g. gadolinium) and superparamagnetic metal (e.g. iron oxide) chelates or particles that modify the relaxation rates of protons in surrounding water molecules. However, the use of such contrast agents is unfortunately hampered by several limitations in terms of specificity, such as indirect detection of water protons through Magnetic resonance (MR) signals, difficulty of quantification that requires image processing, and potential toxicity of the metals. In fact, because of the low sensitivity of MRI, high concentrations of potentially toxic metals is usually required.

KR101836461 describes a Gd-complex of DO3A-ferrocene conjugates as MRI contrast agent. CN101711880 describes the preparation and application of superparamagnetic carbon nanotube contrast agent.

On the other hand, contrast agents that incorporate MRI responsive atoms such as ¹³C, ²H (deuterium), ³¹P, or ¹⁹F, also called MRI probes, can be directly detected, which warrants signal specificity and ease of quantification. In the context of *in vivo* quantitative imaging, ¹⁹F is a very promising nucleus. It benefits from spin ½, 100% natural abundance, high nuclear receptivity, and low background noise as virtually no endogenous fluorine is found in living bodies, as reported by Knight et al. (RSC Adv., 2011, 1, 1415), Tirotta et al. (Chem. Rev., 2015, 115, 1106) et Cho et al. (Bioconjugate Chem., 2019, 30, 2502).

¹⁹F-MRI probes have already been described, as reported by Ahrens et al. (Nat. Biotechnol., 2005, 23, 983). For instance, Jirak et al. (Magn. Reson. Mater. Phys., Biol. Med., 2019, 32, 173) describes the molecule 1,3-bis[[1,1,1,3,3,3-hexafluoro-2-(trifluoromethyl)propan-2-yl]oxy]-2,2-bis[[1,1,1,3,3,3-hexafluoro-2-(trifluoromethyl)propan-2-yl]oxymethyl]propane (PERFECTA), as a ¹⁹F-MRI probe, which is a symmetrical molecule bearing 36 fluorine atoms and one of the few fluorine-based probes. Tirotta et al. (J. Am. Chem. Soc., 2014, 136, 8524) teaches that the chemically equivalent fluorine atoms of PERFECTA resonate at the same frequency and provide a strong and unique single NMR signal, which is suitable for MRI applications. However, the main hindrance to the biological use and *in vivo* applications of PERFECTA is its poor solubility in aqueous media.

Jamgotchian et al. (Nanoscale 2021, 13, 2373-2377) describes nanometric carrier systems, in particular perfluorinated micelles, for the encapsulation and biocompatibilization of PERFECTA and the application to the *in vivo* targeting of solid tumors. However, preclinical imaging performances of such micelles is hampered by some non-specific accumulation of micelles in the liver of a mice model. This resulted in a strong MRI signal in the abdomen, reducing the contrast in the area of interest, in particular the tumor.

Yet, there remains a need for developing solutions and methods for improving the contrast of magnetic resonance imaging and which are biocompatible, in particular with limited or even without toxicity as well as good solubility in aqueous media.

In particular, there remains a need for novel formulations which provide, as an advantage, a high and selective contrast during MRI acquisition between healthy and pathological tissues and therefore exploitable imaging data.

The invention has for purpose to meet the above-mentioned needs.

### SUMMARY OF THE INVENTION

According to a **first main embodiment,** the invention relates to a nanovector comprising:
- an external layer comprising a plurality of amphiphilic molecules, said amphiphilic molecules comprising at least one hydrophobic moiety linked to at least one hydrophilic moiety, and
- a core comprising at least one organometallic complex having a paramagnetic property that can be activated by oxidation, said organometallic complex being functionalized by at least one hydrophobic moiety,
the oxidation of the organometallic complex being controllable by light, the light having in particular a wavelength comprised between 300 nm and 1200 nm, more particularly between 400 and 800 nm, even more particularly between 400 nm and 700 nm.

With the aim of alleviating the lack of contrast during MRI of zones of interest, the inventor has developed a nanovector, which is able to modulate the MRI signal of some specific and selected zones, with full spatial and temporal control, by the application of an external stimulus such as light. More precisely, the nanovector according to the invention is able to turn-off the MRI signal (also called "OFF state") when the organometallic complex is oxidized and has thus paramagnetic property.

Some prior art documents relate to systems presenting a MRI signal which can be modified by using, for example, modulators such as transition metals (e.g. copper, iron) or rare earth (e.g. gadolinium) complexes, some of them incorporating ¹⁹F-MRI probes. In particular, the reported systems can be sensitive to internal (endogenous) stimuli, such as redox potential as teaches by Svec et al. (Macromolecules 2022, 55, 2, 658), pH as teaches by Janasik et al. (ACS Sens. 2023, 8, 2, 721) or US2014/0044648, or enzyme activity as teaches by Cai et al. (Mol. Pharmaceutics 2014, 11, 4208).

However, to the knowledge of the inventors, none of the systems described in these documents can be externally controlled to enhance or decrease the MRI signal.

Furthermore, as opposed to "activation" strategies where the signal can be amplified in the target tissue ("turn-on probe"), the "extinction" strategy ("turn-off probe") as proposed in the present invention is of particular interest in the case of investigative imaging, *i.e.* searching for potential pathological sites.

More precisely, when the nanovector is present in zones not to be imaged by MRI, such as health tissues, the MRI signal relative to such zones can be decreased or even extinguished by the application of light in conditions such as light intensity, wavelength and irradiation time, suitable for inducing oxidation of the organometallic complex. In contrast, the signal relative to the zone of interest, in particular pathological areas such as tumors, is not impacted if no such light is applied. The decrease or extinction of the MRI signal in zones not to be imaged by MRI thus allows to obtain more contrasted images of the zone of interest.

Moreover, considering *in vivo* applications, the signal extinction mediated by the nanovector, in zone(s) not to be imaged by MRI, can be reliably triggered on the basis of the known anatomical location of various peripheral organs, such as the liver, where non-specific accumulation of nanovectors may occur. In such conditions, the enhancement of contrast can be obtained for the tumor to be imaged by MRI.

According to a **second main embodiment,** the invention relates to a composition, comprising water and a plurality of nanovectors as defined above, the amphiphilic molecules being in particular present at a concentration superior or equal to the critical micelle concentration of said amphiphilic molecules, more particularly the composition comprising at least 0,1 g/L of amphiphilic molecules, even more particularly from 5 g/L to 50 g/L of amphiphilic molecules.

According to a **third main embodiment,** the invention relates to a pharmaceutical or diagnostic composition, comprising a plurality of nanovectors as defined above and at least one pharmaceutically acceptable excipient.

According to a **fourth main embodiment,** the invention relates to a method for preparing nanovectors, in particular as defined above, comprising at least the steps of:
a) providing an aqueous composition comprising:
   - a plurality of amphiphilic molecules, said amphiphilic molecules comprising at least one hydrophobic moiety linked to at least one hydrophilic moiety, in particular as defined below;
   - at least one organometallic complex having a paramagnetic property that can be activated by oxidation, said organometallic complex being functionalized by at least one hydrophobic moiety, in particular as defined below;
   - optionally at least one near infrared-responsive photosensitizer suitable to produce oxidizing species when exposed to near infrared light and being functionalized by at least one hydrophobic moiety, in particular as defined below; and
   - optionally at least one probe for magnetic resonance imaging, in particular at least one fluorinated probe for ¹⁹F magnetic resonance imaging, more particularly as defined below; and
b) mixing, in particular ultra-sonicating, the aqueous composition of step a);
   thereby preparing the nanovectors.

According to a **fifth main embodiment,** the invention relates to a nanovector as defined above, a composition as defined above, or a pharmaceutical or diagnostic composition as defined above, for use in *in vivo* diagnostics.

According to a **sixth main embodiment,** the invention relates to a nanovector according to the present invention, a composition according to the present invention, or a pharmaceutical or diagnostic composition according to the present invention, for use in a method of magnetic resonance imaging, in particular ¹⁹F magnetic resonance imaging.

According to a **seventh main embodiment,** the invention relates to a method of magnetic resonance imaging also called MRI, in particular ¹H-MRI, ¹⁹F-MRI, ¹³C-MRI, ²H-MRI or ³¹P-MRI, more particularly ¹⁹F-MRI, comprising at least the steps of:
i) exposing to light, in particular blue light and/or near infrared light, more particularly at a wavelength comprised between 300 nm and 1200 nm, even more particularly between 400 nm and 800 nm, for example between 400 nm and 700 nm, zone(s) of a patient previously administered with nanovector(s) as defined above, in particular with a composition as defined above or a pharmaceutical or diagnostic composition as defined above, said zone(s) being not to be imaged by MRI; and
ii) imaging zone(s) of interest of said patient by MRI, in particular ¹H-MRI, ¹⁹F-MRI, ¹³C-MRI, ²H-MRI or ³¹P-MRI, more particularly ¹⁹F-MRI, the zone(s) of steps i) and ii) being distinct,
   step ii) being consecutive or simultaneous with step i).

According to an **eighth main embodiment,** the invention relates to a kit for magnetic resonance imaging, in particular for ¹H-MRI, ¹⁹F-MRI, ¹³C-MRI, ²H-MRI or ³¹P-MRI, more particularly for ¹⁹F-MRI, comprising:
- a plurality of amphiphilic molecules, said amphiphilic molecules comprising at least one hydrophobic moiety linked to at least one hydrophilic moiety, in particular as defined below;
- at least one organometallic complex having a paramagnetic property that can be activated by oxidation, said organometallic complex being functionalized by at least one hydrophobic moiety, in particular as defined below;
- optionally at least one near infrared-responsive photosensitizer suitable to produce oxidizing species when exposed to near infrared light and being functionalized by at least one hydrophobic moiety, in particular as defined below; and
- optionally at least one probe for magnetic resonance imaging, in particular at least one fluorinated probe for ¹⁹F magnetic resonance imaging, more particularly as defined below.

### BRIEF DESCRIPTION OF THE FIGURES

**Fig. 1****:** Scheme of **PERFECTA/FFc@PFTD-PEG nanovectors** - **formulation # 3** of example 2 (blue light responsive system).
**Fig. 2****:** Scheme of **PERFECTA/FFc/NIR-PS@PFTD-PEG nanovectors** - **formulation # 4** of example 2 (NIR light responsive system).
**Fig. 3****:** Determination of the critical micelle concentration of **PFTD-PEG** (I') as detailed in example 1.
**Fig. 4****:** Dynamic light scattering profile of **PFTD-PEG** (I') **micelles** of example 1.
**Fig. 5****:** Dynamic light scattering profile of **PERFECTA@PFTD-PEG nanovectors** - **formulation # 2** of example 2.
**Fig. 6****:** Dynamic light scattering profile of **PERFECTA/FFc@PFTD-PEG nanovectors** - **formulation # 3** of example 2, plain lines relate to the DLS profile of the nanovectors before irradiation ("on" state) and dashed lines relate to the DLS profile of the same nanovectors after irradiation ("off" state)
**Fig. 7****:** Study of proliferation/survival of MCF7 cell lines treated for 72 h with different formulations of **PFTD-PEG micelles, PERFECTA/FFc@PFTD-PEG nanovectors** and **FFc@PFTD-PEG nanovectors,** at different concentrations: 0, 5, 10, 50, 100 and 500 µM, as detailed in example 3.
**Fig. 8****:** Study of proliferation/survival of MC-38 cell lines treated for 72 h with different formulations of **PFTD-PEG micelles, PERFECTA/FFc@PFTD-PEG nanovectors** and **FFc@PFTD-PEG nanovectors,** at different concentrations: 0, 5, 10, 50, 100 and 500 µM, as detailed in example 3.
**Fig. 9****:** Study of proliferation/survival of MCF7 cells treated with micelles for 72h and Illuminated with blue light (450 nm) for 10 min with different formulations of **PFTD-PEG micelles, PERFECTA/FFc@PFTD-PEG nanovectors** and **FFc@PFTD-PEG nanovectors,** at different concentrations: 0, 5, 10, 50, 100 and 500 µM, as detailed in example 3.
**Fig. 10****:** ¹H-MRI signal modulation study for formulation comprising **PERFECTA/FFc@PFTD-PEG nanovectors** at different concentrations of **FFc (II')** without ("¹H MRI ON") or with ("¹H MRI OFF") illumination for 10 min with blue light (450 nm), and for formulation comprising **PERFECTA/FFc/NIR-PS@PFTD-PEG nanovectors** without ("¹H MRI ON") or with ("¹H MRI OFF") illumination for 10 min with red light (650 nm), as detailed in example 4.
**Fig. 11****:** ¹⁹F-MRI signal modulation study for formulation comprising **PERFECTA/FFc@PFTD-PEG nanovectors** at different concentrations of FFc (II') without ("¹⁹F MRI ON") or with ("¹⁹F MRI OFF") illumination for 10 min with blue light (450 nm), and for formulation comprising **PERFECTA/FFc/NIR-PS@PFTD-PEG nanovectors** without ("¹⁹F MRI ON") or with ("¹⁹F MRI OFF") illumination for 10 min with red light (650 nm), as detailed in example 4.

### DETAILED DESCRIPTION

According to a **first main embodiment,** the invention relates to a nanovector comprising:
- an external layer comprising a plurality of amphiphilic molecules, said amphiphilic molecules comprising at least one hydrophobic moiety linked to at least one hydrophilic moiety, and
- a core comprising at least one organometallic complex having a paramagnetic property that can be activated by oxidation, said organometallic complex being functionalized by at least one hydrophobic moiety,
the oxidation of the organometallic complex being controllable by light, the light having in particular a wavelength comprised between 300 nm and 1200 nm, more particularly between 400 and 800 nm, even more particularly between 400 nm and 700 nm.

In particular, the oxidation of the organometallic complex is controllable directly or indirectly by light. In other word, light, at a specific wavelength, can directly trigger the oxidation of the organometallic complex or can indirectly trigger the oxidation of the organometallic complex via a further compound present in the core and being sensitive to said light.

In particular,
- the organometallic complex is photo-responsive; and/or
- said core further comprises at least one near infrared-responsive photosensitizer suitable to produce oxidizing species when exposed to near infrared light and being functionalized by at least one hydrophobic moiety, in particular as defined below.

When the organometallic complex is photo-responsive, the oxidation of the organometallic complex can be controlled by exposition to light at a wavelength corresponding to the absorption band of the photo-responsive organometallic complex or one of the absorption bands of the photo-responsive organometallic complex, in particular at a wavelength corresponding to the absorption band of the photo-responsive organometallic complex.

When said core further comprises at least one near infrared-responsive photosensitizer, the oxidation of the organometallic complex can be controlled by exposition to light at a wavelength corresponding to the absorption band of the near infrared-responsive photosensitizer or one of the absorption bands of the near infrared-responsive photosensitizer, in particular at a wavelength corresponding to one of the absorption bands of the near infrared-responsive photosensitizer.

It is to be understood that the above-mentioned organometallic complex is not covalently bonded to the amphiphilic molecule(s). In particular, the organometallic complex is distinct from an amphiphilic molecule.

Preferably, the nanovector according to the invention is a micelle. In particular, such micelle retains its integrity, in other terms does not disintegrate, according to pH variation or enzymatic activity.

In particular, the core is inside the external layer. In particular, the external layer is a shell comprising, in particular consisting of, said plurality of amphiphilic molecules, encapsulating the core.

In particular, the hydrophilic moiety of the amphiphilic molecules is directed towards the external surface of the nanovector, while the hydrophobic moiety is directed towards the core of the nanovector.

Advantageously and without wishing to be bound by the theory, it is proposed that the amphiphilic molecules form a hydrophobic space at the core of nanovectors, which can be used as a reservoir for the organometallic complex as described below but also other hydrophobic compounds, such as MRI probes, in particular as described below or near infrared-responsive photosensitizer as described below.

According to a particular embodiment, the nanovector has an average hydrodynamic diameter equal or inferior to 40 nm, in particular ranging from 10 nm to 30 nm, more particularly ranging from 15 nm to 25 nm.

Preferably, the nanovector is biocompatible.

### Amphiphilic molecules

The amphiphilic molecules can be linear or ramified molecules. In particular, they can be linear.

In particular, said amphiphilic molecules can have a molecular weight of at least 500 Daltons, in particular a molecular weight of at least 1000 Daltons, more particularly a molecular weight of at least 1500 Daltons, preferably a molecular weight of at least 2000 Daltons.

In particular, said amphiphilic molecules can have a molecular weight of less than or equal to 20000 Daltons, in particular a molecular weight of less than or equal to 10000 Daltons, more particularly a molecular weight of less than or equal to 5000 Daltons, preferably a molecular weight of less than or equal to 3000 Daltons.

In particular, said amphiphilic molecules can have a molecular weight comprised between 1000 Daltons and 20000 Daltons, in particular between 1000 Daltons and 10000 Daltons, more particularly between 1000 Daltons and 5000 Daltons, preferably between 1000 Daltons and 3000 Daltons.

According to some embodiments, the nanovectors may be characterized in that the amphiphilic molecules have a molecular weight of at least 500 Daltons, and the nanovector has an average hydrodynamic diameter equal or inferior to 40 nm, in particular in that the amphiphilic molecules have a molecular weight comprised between 1000 Daltons and 20000 Daltons, and the nanovector has an average hydrodynamic diameter comprised between 5 nm and 40 nm, more particularly in that the amphiphilic molecules have a molecular weight comprised between 1500 Daltons and 5000 Daltons, and the nanovector has an average hydrodynamic diameter comprised between 10 nm and 30 nm, even more particularly in that the amphiphilic molecules have a molecular weight comprised between 2000 Daltons and 3000 Daltons, and the nanovector has an average hydrodynamic diameter comprised between 15 nm and 25 nm.

In particular, examples of such amphiphilic molecules have been described in WO2024/121267.

### hydrophilic moiety

According to a particular embodiment, the nanovector may be characterized in that the hydrophilic moiety comprises a polymer, such as a homopolymer or a heteropolymer, in particular a polyoxygenated polymer, and/or a polymer selected from: synthetic polymers, natural polymers and zwitterionic polymers; and more particularly a polymer selected from the group consisting of: polyethylene glycol (PEG), poly(oligo(ethylene glycol) methyl ether methacrylate (POEGMA), polyglycerols (PGs), poly(oxazolines) (POX), poly(hydroxypropylmethacrylate) (PHPMA), poly(2-hydroxyethylmethacrylate) (PHEMA), poly(N-(2-hydroxypropyl)methacrylamide) (HPMA), poly(vinylpyrrolidone) (PVP), poly(N,N-dimethyl acrylamide) (PDMA), and poly(N-acryloylmorpholine) (PAcM), poly(acrylamide), poly(methacrylamide), poly(N-acryloylmorpholine), glycosaminoglycan (GAGs), polyhyaluronic acid (pHA), polyheparin, polysialic acid (pSA), polyaminoacid, polypeptide, poly(carboxybetaine acrylamide) (pCBAA), poly(carboxybetaine methacrylate), poly(sulfobetaine methacrylate), poly(methacryloyloxyethylphosphorylcholine), poly(vinyl-pyridiniopropanesulfonate), poly(carboxybetaine) (pCB), poly(sulfobetaine) (pSB), phosphobetaine-based, poly(2-methacryloyloxyethylphosphorylcholine) (PMPC) and poly(β-amino ester) (PAE) based polymers, even more particularly a polymer selected from the group consisting of: polyethylene glycol (PEG), poly(vinylpyrrolidone) (PVP), polyglycerols (PGs) and poly(vinyl-pyridiniopropanesulfonate).

Advantageously, the hydrophilic moiety can comprise, or even consist of, polyethylene glycol (PEG). Without wishing to be bound by the theory, the inventors are of the opinion that when the external surface of the nanovector is pegylated, it provides to the nanovectors with a stealth character characterized by the avoidance of the capture by immune system, allowing a prolonged residence time in the bloodstream and facilitating their passive accumulation within pathological tissues, in particular within tumors through the EPR (Enhanced Permeability and Retention) effect.

According to a particular embodiment, the nanovector may be characterized in that the hydrophilic moiety comprises or consists of a polyoxygenated polymer, more particularly a polyethylene glycol (PEG) polymer, for example a polyethylene glycol (PEG) polymer having a molecular weight of at least 350 Daltons, for example a polyethylene glycol (PEG) polymer having a molecular weight of at least 500 Daltons, for example a polyethylene glycol (PEG) polymer having a molecular weight of at least 1000 Daltons, for example a polyethylene glycol (PEG) polymer having a molecular weight of at least 1500 Daltons, for example a polyethylene glycol (PEG) polymer having a molecular weight of at least 2000 Daltons.

According to a particular embodiment, the nanovector may be characterized in that the hydrophilic moiety comprises or consists of a polyethylene glycol (PEG) polymer having a molecular weight comprised between 350 Daltons and 15000 Daltons, for example comprised between 500 Daltons and 5000 Daltons, for example comprised between 1000 Daltons and 3000 Daltons, for example comprised between 1500 Daltons and 2500 Daltons.

According to a particular embodiment, the nanovector may be characterized in that the hydrophilic moiety comprises a polyethylene glycol (PEG) containing at least 8 ethoxy monomers; for example at least 9, 10, 11, 12, 13, 14, 15, 16, 17, 18, 19, 20, 21, 22, 23, 24, 25, 26, 27, 28, 29, 30, 40, 41, 42, 43, 44, 45, 46, 47, 48, 49, 50 ethoxy monomers.

According to a particular embodiment, the nanovector may be characterized in that the hydrophilic moiety comprises a polyethylene glycol (PEG) containing 100 ethoxy monomers or less than 100 ethoxy monomers; for example less than 99, 98, 97, 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50 ethoxy monomers.

According to a particular embodiment, the nanovector may be characterized in that the hydrophilic moiety comprises a polyethylene glycol (PEG) containing between 8 and 100 ethoxy monomers, in particular containing between 30 and 60 ethoxy monomers, and more particularly containing between 40 and 50 ethoxy monomers.

### hydrophobic moiety

The hydrophobic moiety may be a linear or ramified hydrophobic moiety, more particularly a linear hydrophobic moiety.

According to a particular embodiment, the hydrophobic moiety, linked to at least one hydrophilic moiety, comprises or consists of at least one moiety selected from: a substituted (C₁-C₃₀) alkyl moiety or unsubstituted (C₁-C₃₀) alkyl moiety.

According to exemplified embodiments, the hydrophobic moiety, linked to at least one hydrophilic moiety, comprises or consists of at least one moiety selected from: a linear substituted (C₁-C₃₀)alkyl moiety or a linear unsubstituted (C₁-C₃₀)alkyl moiety.

According to a particular embodiment, the amphiphilic molecules comprise at least one hydrophobic perfluorinated moiety linked to the hydrophilic moiety. In particular, the perfluorinated hydrophobic moiety is linear or ramified, more particularly linear.

In particular, the hydrophobic perfluorinated moiety comprises or consists of at least one moiety selected from: a perfluorinated substituted (C₁-C₃₀)alkyl moiety or perfluorinated unsubstituted (C₁-C₃₀) alkyl moiety.

In particular, the hydrophobic perfluorinated moiety comprises or consists of at least one moiety selected from: a linear perfluorinated substituted (C₁-C₃₀)alkyl moiety or a linear perfluorinated unsubstituted (C₁-C₃₀)alkyl moiety.

In particular, the hydrophobic moiety and the hydrophilic moiety may be linked via at least one linker region. Examples of such linker regions may consist of those selected from: single bonds, carbonyl groups, alkyl groups, -O-, -O(CH₂)ₑ-, -(CH₂)ₑO-, -(CH₂)ₑ-, - (CH₂)ₑO(CH₂)-, -(CH₂)ₑNR¹¹(CH₂)_{f}, -(CH₂)ₑOCONR¹²(CH₂)_{f}-, -(CH₂)ₑCONR¹³(CH₂)_{f}-, - (CH₂)ₑNR¹⁴COO(CH₂)_{f}-, -(CH₂)ₑNR¹⁵CO(CH₂)- or -(CH₂)ₑNR¹⁶CONR¹⁷(CH₂)- ; where each of R¹¹-R¹⁷ is independently hydrogen, methyl, or C₁-C₅ alkyl; and where each of e and f is independently an integer selected from the range of 0 to 5. In some embodiments, the linking moiety is -(CH₂)ₑO(CH₂)_{f} -, where e and f are each independently 1.

Examples of functional groups which may act as linker include those selected from: alkyl, alkenyl, alkynyl, alkylamides, carboxylic esters, carbonates, carbamates, carbamides, amides, sulfamides, sulfides, disulfides, sulfonic esters, phosphoric esters, ketone.

According to a particular embodiment, the nanovector may be characterized in that the plurality of amphiphilic molecules comprises or consists of:

[C] - [Linker]ᵥ - [D];

or

[C]ₜₑᵣₘ - [C] - [Linker1]ᵥ - [D] - [D]ₜₑᵣₘ;

[C]ₜₑᵣₘ - [Linker1]ᵥ - [C] - [Linker2]_{w} - [D] - [Linker3]_{z} - [D]ₜₑᵣₘ;

wherein v and w and z are, independently, 0 or 1;
wherein [C] is a perfluorinated hydrophobic moiety, in particular a substituted or unsubstituted perfluorinated (C₁-C₃₀)alkyl moiety;
wherein [C]ₜₑᵣₘ is a hydrophobic terminal moiety, in particular a perfluorinated hydrophobic moiety, for example selected from: -CF₃ or -RCF₃ with R being a (C₁-C₃₀)alkyl group;
wherein [D] is a hydrophilic moiety, in particular a hydrophilic polymer, for example a polyethylene glycol (PEG) polymer;
wherein [D]ₜₑᵣₘ is a hydrophilic terminal moiety;
wherein [Linker1] and [Linker2] and [Linker3] are the same linker regions or different linker regions; for example linker regions comprising or consisting of a substituted or unsubstitued alkyl (e.g. a (C₁-C₃₀)alkyl group) or a substituted or unsubstituted alkoxy (e.g. a (C₁-C₃₀)alkoxy group).

According to particular embodiments, [D]ₜₑᵣₘ is selected from an hydroxy-terminated or alkoxy-terminated or amino-terminated or siloxane-terminated functional group, in particular comprises an alkoxy-terminated functional group, for instance a methoxy-terminatd functional group.

According to particular embodiments, [D]ₜₑᵣₘ may be selected from a functional group comprising or consisting of: -OH or -OCH₃ or -COCH₃ or -COOH or -CH₂COOH or -NH₃, in particular-OCH₃ or -COCH₃.

According to a particular embodiment, the nanovector may be characterized in that the amphiphilic molecule is a block copolymer, a di-block copolymer or a tri-block copolymer, comprising at least one hydrophobic perfluorinated block moiety linked to at least one hydrophilic block moiety.

According to a particular embodiment, the nanovector may be characterized in that the plurality of amphiphilic molecules comprises, in particular consists of, compounds of formula (I): 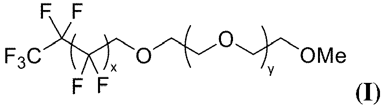
wherein x is equal or superior to 1;
wherein y is equal or superior to 1;
with "Me" referring to a methyl (-CH₃),
or mixtures of compounds of formula (I).

According to particular embodiments of formula (I), x is equal or superior to 8 and y is equal or superior to 8.

According to particular embodiments of formula (I), x ranges from 1 to 100 and y ranges from 1 to 100.

According to particular embodiments of formula (I), the ratio x/y is equal or inferior to 1, with x and y being separately equal or superior to 1, for example equal or superior to 8.

According to a particular embodiment, the nanovector may be characterized in that the plurality of amphiphilic molecules comprises, in particular consists of, compounds of formula (I): 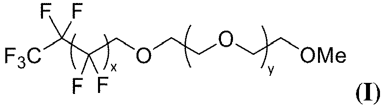
wherein x ranges from 8 to 15;
wherein y ranges from 8 to 100,
or mixtures of compounds of formula (I).

According to a particular embodiment, the nanovector may be characterized in that the plurality of amphiphilic molecules comprises, in particular consists of, compounds of formula (I'):

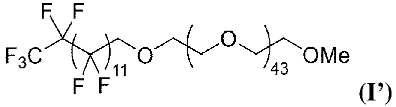

### Organometallic complex

In particular, the organometallic complex is photo-responsive, in particular undergo oxidation upon light illumination at one or more wavelengths, corresponding to the absorption band of the organometallic complex or one of the absorption bands of the organometallic complex, comprised between 300 nm and 800 nm, more particularly between 380 nm and 520 nm, even more particularly between 400 nm and 500 nm.

According to a particular embodiment, the paramagnetic property of the organometallic complex can be activated by:
- oxidation directly induced by light, in particular at a wavelength comprised between 300 nm and 800 nm, more particularly between 380 nm and 520 nm, even more particularly between 400 nm and 500 nm, or
- induced by oxidizing species, in particular singlet oxygen (¹O₂), hydrogen peroxide (H₂O₂), ozone and/or dibenzoyl peroxide, more particularly singlet oxygen (¹O₂).

In particular, the organometallic complex has diamagnetic property when paramagnetic property is not activated.

In particular, the organometallic complex comprises at least one metal chosen from transition metals, in particular from iron, copper and manganese.

According to a particular embodiment, the nanovector may be characterized in that the organometallic complex comprises at least one metallocene group, optionally functionalized by at least one hydrophobic perfluorinated moiety.

Advantageously, the organometallic complex comprises at least one ferrocenyl group, optionally functionalized by at least one hydrophobic perfluorinated moiety.

Without wishing to be bound by the theory, it is proposed that the organometallic complex comprising at least one ferrocenyl group, in particular as described below, can undergo oxidation upon, for example, blue light illumination or singlet oxygen (¹O₂). In the absence of light or ¹O₂, the iron of the ferrocene group is in its iron(II) state and does not disturb the MRI signal. This condition corresponds to a "ON state" of the system that can readily be visualized by MRI. On the other hand, when ferrocene group is oxidized, for instance by application of light or by the presence of ¹O₂, the iron of the ferrocene group is oxidized into paramagnetic iron(III), which drastically extinguishes the MRI signal. This condition corresponds to the "OFF state" of the system which can hardly or even no longer be visualized by MRI because of the presence of the paramagnetic iron.

In particular, the organometallic complex is a MRI signal modulator.

According to a particular embodiment, the organometallic complex is a compound of formula (II): 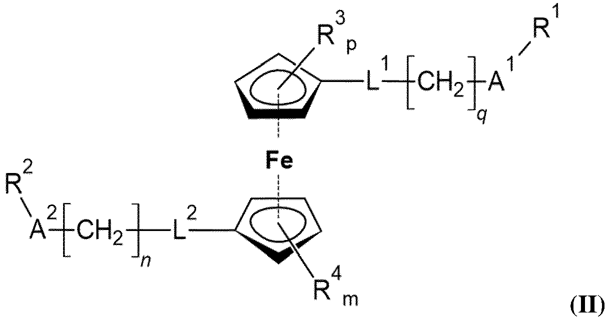 wherein:
- L¹ and L² independently represent a single bond, a -O- group, a -NH- group, a -C(=O)-group, a -C(=O)-O- group, a -O-C(=O)- group, a -NH-C(=O)- group, or a -C(=O)-NH-group, in particular L¹ and L² independently represent a -C(=O)-O- group being linked to said ferrocenyl group via its carbon atom and to the group [CH₂]_{q} or [CH₂]ₙ via the oxygen atom, or a -C(=O)-NH- group being linked to said ferrocenyl group via its carbon atom and to the group [CH₂]_{q} or [CH₂]ₙ via its nitrogen atom;
- n and q are independently equal to 0, 1, 2, 3 or 4;
- A¹ and A² independently represent a single bond, a -O- group, a -NH- group, a -C(=O)-group, a -C(=O)-O- group, a -O-C(=O)- group, a -C(=O)-NH- group, or a -NH-C(=O)-group, in particular A¹ and A² independently represent a -C(=O)-O- group, a -O-C(=O)-group, a -NH-C(=O)- group, or a -C(=O)-NH- group;
- R¹ and R² independently represent a hydrophobic moiety selected from a substituted or unsubstituted, saturated or unsaturated, aliphatic C₄-C₃₆ chain-containing moiety and a hydrophobic perfluorinated moiety, in particular a hydrophobic perfluorinated moiety, more particularly a -(CF₂)ₜ-CF₃ group wherein t is equal to 1 to 25;
- p and m are identical or different, and equal to 0, 1, 2 or 3; and
- when p and/or m is(are) strictly greater than 0, R³ and R⁴ independently represent a (C₁-C₆)alkyl group, a (C₂-C₆)alkenyl group, a (C₅-C₁₁)aryl group, a (C₃-C₈)cycloalkyl group, a (C₅-C₁₁)heteroaryl, (C₃-C₈)heterocycloalkyl group or a halogen atom, in particular selected from an iodine atom, a chlorine atom and a bromine atom,
or a mixture of compounds of formula (II).

According to a particular embodiment of formula (II),
- L¹ and L² independently represent a -C(=O)-O- group being linked to said ferrocenyl group via its carbon atom and to the group [CH₂]_{q} or [CH₂]ₙ via the oxygen atom, or a - C(=O)-NH- group being linked to said ferrocenyl group via its carbon atom and to the group [CH₂]_{q} or [CH₂]ₙ via its nitrogen atom;
- n and q are independently equal to 1, 2 or 3;
- A¹ and A² independently represent a -C(=O)- group, a -C(=O)-O- group, a -O-C(=O)-group, a -C(=O)-NH- group, or a -NH-C(=O)- group, in particular a -C(=O)- group, a - C(=O)-NH- group, or a -NH-C(=O)- group;
- R¹ and R² independently represent a hydrophobic perfluorinated moiety, in particular a -(CF₂)ₜ-CF₃ group wherein t is equal to 1 to 25, more particularly equal to 3 to 15, even more particularly equal to 5 to 10 and for example equal to 6;
- p and m are independently equal to 0 or 1, in particular equal to 0; and
- when p and/or m is(are) equal to 1, R³ and R⁴ independently represent a halogen atom, for example selected from an iodine atom, a chlorine atom and a bromine atom,

According to a particular embodiment of formula (II), p and m are equal to 0.

According to a particular embodiment of formula (II), A¹ and A² independently represent a -C(=O)- group, a -C(=O)-NH- group, or a -NH-C(=O)- group.

According to a particular embodiment of formula (II), L¹ and L² represent a -C(=O)-NH- group being linked to said ferrocenyl group via its carbon atom and to the group [CH₂]_{q} or [CH₂]ₙ via its nitrogen atom.

According to a particular embodiment of formula (**II**), R¹ and R² independently represent a -(CF₂)ₜ-CF₃ group wherein t is equal to 3 to 15, in particular equal to 5 to 10 and for example equal to 6.

According to a particular embodiment of formula (**II**),
- L¹ and L² represent a -C(=O)-NH- group being linked to said ferrocenyl group via its carbon atom and to the group [CH₂]_{q} or [CH₂]ₙ via its nitrogen atom; and
- A¹ and A² independently represent a -C(=O)- group, a -C(=O)-NH- group, or a -NH-C(=O)- group.

According to a particular embodiment of formula (**II**),
- R¹ and R² independently represent a -(CF₂)ₜ-CF₃ group wherein t is equal to 3 to 15, in particular equal to 5 to 10 and for example equal to 6;
- p and m are independently equal to 0 or 1, in particular equal to 0; and
- when p and/or m is(are) equal to 1, R³ and R⁴ independently represent a halogen atom, for example selected from an iodine atom, a chlorine atom and a bromine atom.

According to a particular embodiment of formula (**II**),
- L¹ and L² represent a -C(=O)-NH- group being linked to said ferrocenyl group via its carbon atom and to the group [CH₂]_{q} or [CH₂]ₙ via its nitrogen atom;
- n and q are independently equal to 1, 2 or 3;
- A¹ and A² independently represent a -C(=O)- group, a -C(=O)-NH- group, or a -NH-C(=O)- group;
- R¹ and R² independently represent a -(CF₂)ₜ-CF₃ group wherein t is equal to 3 to 15, in particular equal to 5 to 10 and for example equal to 6;
- p and m are independently equal to 0 or 1, in particular equal to 0; and
- when p and/or m is(are) equal to 1, R³ and R⁴ independently represent a halogen atom, for example selected from an iodine atom, a chlorine atom and a bromine atom.

According to a preferred embodiment, the organometallic complex comprises at least, notably consists of, the compound of formula (**II'**):

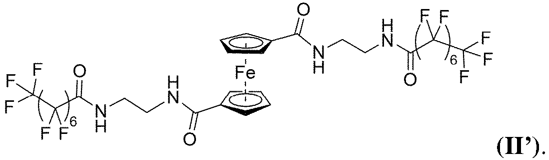

According to a particular embodiment, the organometallic complex is present in the nanovector in an amount from 1% to 50% by weight, in particular from 5% to 40 % by weight, and more particularly from 10% to 30% by weight, relative to the total weight of the nanovector.

In particular, the nanovector according to the invention comprises:
- an external layer comprising a plurality of amphiphilic molecules having a hydrophilic moiety comprising a polyethylene glycol (PEG), in particular comprising, notably consisting of, compounds of formula (**I**) as defined above or mixtures of compounds of formula (**I**) as defined above, more particularly compounds of formula (**I**') as defined above, and
- a core comprising at least one organometallic complex comprising at least one ferrocenyl group, in particular being a compound of formula (**II**) as defined above or a mixture of compounds of formula (**II**) as defined above, more particularly of formula (**II**') as defined above.

In particular, the nanovector according to the invention comprises:
- an external layer comprising a plurality of amphiphilic molecules comprising, in particular consisting of, compounds of formula (**I)** as defined above or mixtures of compounds of formula (**I**) as defined above, in particular compounds of formula (**I'**) as defined above and
- a core comprising at least one organometallic complex being a compound of formula (**II**) as defined above or a mixture of compounds of formula (**II**) as defined above, in particular of formula (**II'**) as defined above,
the organometallic complex being present in an amount from 1% to 50% by weight, in particular from 5% to 40 %, and more particularly from 10% to 30% by weight, relative to the total weight of the nanovector.

### Near infrared-responsive photosensitizer

According to a particular embodiment, the core of the nanovector according to the invention further comprises at least one near infrared-responsive photosensitizer suitable to produce oxidizing species when exposed to near infrared light and being functionalized by at least one hydrophobic moiety, in particular at least one hydrophobic perfluorinated moiety.

According to this embodiment, the organometallic complex and the near infrared-responsive photosensitizer are co-encapsulated by the external layer, in particular by a shell comprising, notably consisting of, amphiphilic molecules as described above.

Oxidizing species may be singlet oxygen (¹O₂).

In particular, the near infrared-responsive photosensitizer(s) may have an absorption band equal or superior to 600 nm, in particular ranging from 600 nm to 1200 nm, more particularly from 600 nm to 800 nm, even more particularly from 620 nm to 700 nm. In particular, the near infrared-responsive photosensitizer is suitable to produce oxidizing species when exposed to near infrared light at a wavelength, corresponding to its absorption band or one of its absorption bands, in particular to one of its absorption bands, equal or superior to 600 nm, more particularly ranging from 600 nm to 1200 nm, more particularly from 600 nm to 800 nm, even more particularly from 620 nm to 700 nm.

According to a particular embodiment, the near infrared-responsive photosensitizer comprises in particular at least one group chosen from phenothiazines, porphyrins, chlorins, bacteriochlorins, cyanines, phtalocyanines, pyropheophorbide-a or derivatives thereof.

According to a particular embodiment, the near infrared-responsive photosensitizer comprises at least a group chosen from a pyropheophorbide-a group or a derivative thereof.

According to a particular embodiment, the near infrared-responsive photosensitizer is compound of formula (III): 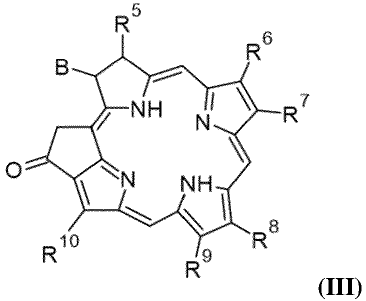 wherein:
- R⁵, R⁶, R⁷, R⁸, R⁹ and R¹⁰ independently represent a hydrogen atom, a (C₁-C₆)alkyl group or a (C₂-C₆)alkenyl group; and
- B represents a hydrophobic moiety, in particular comprising a substituted or unsubstituted, saturated or unsaturated, aliphatic C₄-C₃₆ chain, and/or a hydrophobic perfluorinated moiety,
or a mixture of compounds of formula (III).

According to a particular embodiment of formula (**III**):
- R⁵, R⁶, R⁸, R⁹ and R¹⁰ independently represent a (C₁-C₄)alkyl group;
- R⁷ represents a (C₂-C₄)alkenyl group;
- B comprises a hydrophobic perfluorinated moiety, in particular B represents a -(CH₂)ᵣ-X-(CH₂)ₛ-Y-(CF₂)ₜ-CF₃ group, wherein
   - r and s are independently equal to 0, 1, 2, 3 or 4, in particular equal to 1, 2 or 3,
   - t is equal to 1 to 25, in particular equal to 3 to 15, more particularly equal to 5 to 10 and even more particularly equal to 6, and
- X and Y independently represent a single bond, a -O- group, a -NH- group, a -C(=O)-group, a -C(=O)-O- group, a -O-C(=O)- group, a -NH-C(=O)- group, or a -C(=O)-NH-group, in particular a -C(=O)-O- group, a -O-C(=O)- group, a -NH-C(=O)- group, or a -C(=O)-NH- group.

Preferably, in this particular embodiment, B represents a -(CH₂)ᵣ-X-(CH₂)ₛ-Y-(CF₂)ₜ-CF₃ group,
wherein :
- r and s are independently equal to 0, 1, 2, 3 or 4, in particular equal to 1, 2 or 3,
- t is equal to 1 to 25, in particular equal to 3 to 15, more particularly equal to 5 to 10 and even more particularly equal to 6, and
- X and Y independently represent a single bond, a -O- group, a -NH- group, a - C(=O)- group, a -C(=O)-O- group, a -O-C(=O)- group, a -NH-C(=O)- group, or a - C(=O)-NH- group, in particular a -C(=O)-O- group, a -O-C(=O)- group, a -NH-C(=O)- group, or a -C(=O)-NH- group;

in particular wherein:
   - r and s are independently equal to 1, 2 or 3,
   - t is equal to 1 to 25, in particular equal to 3 to 15, more particularly equal to 5 to 10 and even more particularly equal to 6, and
   - X and Y independently represent a single bond, a -O- group, a -NH- group, a - C(=O)- group, a -C(=O)-O- group, a -O-C(=O)- group, a -NH-C(=O)- group, or a - C(=O)-NH- group, in particular a -C(=O)-O- group, a -O-C(=O)- group, a -NH-C(=O)- group, or a -C(=O)-NH- group;
more particularly wherein:
   - r and s are independently equal to 1, 2 or 3,
   - t is equal to 3 to 15, more particularly equal to 5 to 10 and even more particularly equal to 6, and
   - X and Y independently represent a single bond, a -O- group, a -NH- group, a - C(=O)- group, a -C(=O)-O- group, a -O-C(=O)- group, a -NH-C(=O)- group, or a - C(=O)-NH- group, in particular a -C(=O)-O- group, a -O-C(=O)- group, a -NH-C(=O)- group, or a -C(=O)-NH- group;
even more particularly wherein:
   - r and s are independently equal to 1, 2 or 3,
   - t is equal to 3 to 15, more particularly equal 5 to 10 and even more particularly equal to 6, and
   - X and Y independently represent a -C(=O)- group, a -C(=O)-O- group, a -O-C(=O)- group, a -NH-C(=O)- group, or a -C(=O)-NH- group, in particular a--C(=O)-O- group, a -O-C(=O)- group, a -NH-C(=O)- group, or a -C(=O)-NH-group;
even more particularly wherein:
   - r and s are independently equal to 1, 2 or 3,
   - t is an integer comprised between 3 to 15, more particularly 5 to 10 and even more particularly equal to 6, and
   - X and Y independently represent a -C(=O)-O- group, a -O-C(=O)- group, a -NH-C(=O)- group, or a -C(=O)-NH- group.

According to a particular embodiment, the near infrared-responsive photosensitizer comprises at least, notably consists of, the compound of formula (III'),

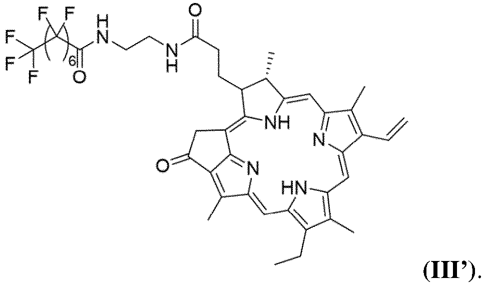

According to a particular embodiment, the near infrared-responsive photosensitizer is present in the nanovector in an amount from 0,1% to 5% by weight, in particular from 0,3% to 2% by weight, relative to the total weight of the nanovector.

According to a particular embodiment, the nanovector according to the invention comprises:
- an external layer comprising a plurality of amphiphilic molecules, said amphiphilic molecules comprising at least one hydrophobic moiety linked to at least one hydrophilic moiety, and
- a core comprising:
   - at least one organometallic complex having a paramagnetic property that can be activated by oxidation, said organometallic complex being functionalized by at least one hydrophobic moiety; and
   - at least one near infrared-responsive photosensitizer suitable to produce oxidizing species when exposed to near infrared light and being functionalized by at least one hydrophobic moiety.

Without wishing to be bound by the theory, it is proposed that, under near infrared illumination at the absorption band or one of the absorption bands of the photosensitizer, in particular at one of the absorption bands of the photosensitizer, near infrared-responsive photosensitizer as defined above, in particular of formula (**III**) as defined above, more particularly of formula (**III'**) as defined above, is excited and produces oxidizing species, in particular singlet oxygen (¹O₂), which, in turn, oxidizes the organometallic complex, in particular of formula (**II**) as defined above, more particularly of formula (**II'**) as defined above, and thus extinguishes the MRI signal. The near infrared-responsive photosensitizer as defined above allows thus to activate the paramagnetic property of the organometallic complex, upon exposition to light at a wavelength corresponding to the absorption band or one of the absorption bands of the near infrared-responsive photosensitizer, thus leading the system to its "OFF state" as mentioned above.

A nanovector according to the invention comprising the organometallic complex and the near infrared-responsive photosensitizer allows the organometallic complex to act as a MRI modulator, under an illumination wavelength corresponding to NIR, defined as the "OFF state" as mentioned above.

According to a particular embodiment, the nanovector according to the invention comprises:
- an external layer comprising a plurality of amphiphilic molecules having a hydrophilic moiety comprising a polyethylene glycol (PEG), in particular comprising, notably consisting of, compounds of formula (**I**) as defined above or mixtures of compounds of formula (**I**) as defined above, more particularly compounds of formula (**I'**) as defined above and
- a core comprising:
   - at least one organometallic complex comprising at least one ferrocenyl group, in particular being a compound of formula (**II**) as defined above or a mixture of compounds of formula (**II**) as defined above, more particularly of formula (**II**') as defined above; and
   - at least one near infrared-responsive photosensitizer as described above, comprising at least a group chosen from a pyropheophorbide-a group or a derivative thereof, in particular being of a compound of formula (**III**) as defined above or a mixture of compounds of formula (**III**) as defined above, more particularly of formula (**III'**) as defined above.

According to a particular embodiment, the nanovector according to the invention comprises:
- an external layer comprising a plurality of amphiphilic molecules comprising, notably consisting of, compounds of formula (**I'**) as defined above or mixtures of compounds of formula (**I**') as defined above, and
- a core comprising:
   - at least one organometallic complex comprising at least, notably consisting of, the compound of formula (**II'**) as defined above; and
   - at least one near infrared-responsive photosensitizer comprising at least, notably consisting of, a compound of formula (**III'**) as defined above.

According to a particular embodiment, the invention also relates to a nanovector comprising:
- an external layer comprising a plurality of amphiphilic molecules comprising, notably consisting of, compounds of formula (**I**) as defined above or mixtures of compounds of formula (**I**) as defined above, in particular compounds of formula (**I**') as defined above and

- a core comprising:
   - at least one organometallic complex being a compound of formula (**II**) as defined above or a mixture of compounds of formula (**II**) as defined above, in particular a compound of formula (**II'**) as defined above; and
   - at least one near infrared-responsive photosensitizer being a compound of formula (**III**) as defined above or a mixture of compounds of formula (**III**) as defined above, in particular a compound of formula (**III'**) as defined above,
the near infrared-responsive photosensitizer being present in an amount from 0,1% to 5% by weight, in particular from 0,3% to 2% by weight, relative to the total weight of the nanovector.

According to a particular embodiment, the invention also relates to a nanovector comprising:
- an external layer comprising a plurality of amphiphilic molecules comprising, notably consisting of, compounds of formula (**I**) as defined above or mixtures of compounds of formula (**I**) as defined above, in particular compounds of formula (**I')** as defined above and
- a core comprising:
   - at least one organometallic complex being a compound of formula (**II**) as defined above or a mixture of compounds of formula (**II**) as defined above, in particular a compound of formula (**II**') as defined above; and
   - at least one near infrared-responsive being compound of formula (**III**) as defined above or a mixture of compounds of formula (**III**) as defined above, in particular a compound of formula (**III**') as defined above,

the organometallic complex being present in an amount from 1% to 50% by weight, in particular from 5% to 40 %, and more particularly from 10% to 30% by weight, relative to the total weight of the nanovector, and
the near infrared-responsive photosensitizer being present in an amount from 0,1% to 5% by weight, in particular from 0,3% to 2% by weight, relative to the total weight of the nanovector.

Advantageously, the above mentioned nanovectors which comprises at least one near infrared-responsive photosensitizer and are thus sensitive to near infrared light can be in particular used for *in vivo* applications, as the window of tissue transparency lies in the NIR region.

### Probe for magnetic resonance imaging

According to a particular embodiment, the core of the nanovector according to the invention further comprises at least one probe for magnetic resonance imaging, also called MRI.

According to this embodiment, the organometallic complex and the probe for magnetic resonance imaging are co-encapsulated by the external layer, in particular by a shell comprising, notably consisting of, amphiphilic molecules as described above.

In particular, the probe for MRI may be chosen among probes for ¹H-MRI, ¹⁹F-MRI, ¹³C-MRI, ²H-MRI or ³¹P-MRI, in particular among probes for ¹⁹F-MRI, ¹³C-MRI, ²H-MRI or ³¹P-MRI.

According to a particular embodiment, the probe for MRI is a fluorinated probe for ¹⁹F magnetic resonance imaging, also called ¹⁹F-MRI.

Advantageously, the fluorinated probe for ¹⁹F-MRI comprises at least 3 chemically equivalent fluorine atoms, in particular at least 9 chemically equivalent fluorine atoms, more particularly at least 18 chemically equivalent fluorine atoms, even more particularly at least 36 chemically equivalent fluorine atoms.

According to a particular embodiment, the fluorinated probe for ¹⁹F-MRI comprises at least one perfluorinated moiety.

According to a particular embodiment, the fluorinated probe for ¹⁹F-MRI comprises a compound chosen from hexafluorobenzene (HFB), perfluorodecalin (PFDC), perfluorononane (PFN), perfluoroctyl bromide (PFOB), perfluoro-15-crown-5-ether (PFCE), perfluoropolyethers (PFPEs), N-(2,2,2-trifluorethyl)acrylate (TFEA), N-(2,2,2-trifluorethyl)methacrylate (TFEMA), 2,3,4,5,6-pentafluorostyrene (PFS), octafluoropentyl methacrylate (OFPMA), N-(2,2-difluorethyl)acrylamide (PDFEA), 1,3-bis[[1,1,1,3,3,3-hexafluoro-2-(trifluoromethyl)propan-2-yl]oxy]-2,2-bis [[1,1,1,3,3,3-hexafluoro-2-(trifluoromethyl)propan-2-yl]oxymethyl]propane (PERFECTA) and mixtures thereof.

According to a particular embodiment, the fluorinated probe for ¹⁹F-MRI comprises at least, notably consists of, the compound PERFECTA of formula (**IV**):

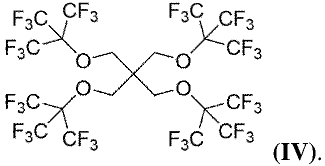

According to a particular embodiment, the probe for magnetic resonance imaging is present in the nanovector in an amount from 1% to 60% by weight, in particular from 10% to 50% by weight, more particularly from 20% to 50% by weight, notably from 30% to 50% by weight, relative to the total weight of the nanovector.

According to a particular embodiment, the fluorinated probe for ¹⁹F magnetic resonance imaging, in particular of formula (**IV**), is present in the nanovector in an amount from 1% to 60% by weight, in particular from 10% to 50% by weight, more particularly from 20% to 50% by weight, notably from 30% to 50% by weight, relative to the total weight of the nanovector.

Without wishing to be bound by the theory, the inventors are of the opinion that the MRI signal emitted by the probe for MRI can be attenuated, or even extinguished, in healthy tissue, by triggering oxidation of the organometallic complex, in particular of formula (**I**I) as defined above, more particularly of formula (**II**') as defined above, which becomes paramagnetic as mentioned above, and locally modifies the relaxivity of the probe for MRI, in particular the probe for ¹⁹F-MRI as defined above, more particularly the probe PERFECTA of formula (**IV**) as defined above. As detailed previously, the oxidation of the organometallic complex may be triggered upon focused illumination of the nanovector at a wavelength corresponding to the absorption wavelength of the organometallic complex when photo-responsive and/or at a wavelength corresponding to the absorption wavelength of a near-infrared responsive photosensitizer when present.

In particular, in the absence of light or ¹O₂, the iron of the ferrocene group of compound of formula (**II'**) is in its iron(II) state and does not disturb the MRI signal of the probe. This condition corresponds to a "ON state" of the probe that can readily be visualized by MRI. On the other hand, when ferrocene group of compound of formula (**II**') is oxidized, for instance by application of light or by the presence of ¹O₂, the iron of the ferrocene group is oxidized into paramagnetic iron(III), which drastically extinguishes the MRI signal of the probe. This condition corresponds to the "OFF state" of the probe, which can hardly or even no longer be visualized by MRI because of the presence of the paramagnetic iron.

According to a particular embodiment, the nanovector according to the invention comprises:
- an external layer comprising a plurality of amphiphilic molecules, said amphiphilic molecules comprising at least one hydrophobic moiety linked to at least one hydrophilic moiety, and
- a core comprising:
   - at least one organometallic complex having a paramagnetic property that can be activated by oxidation, said organometallic complex being functionalized by at least one hydrophobic moiety; and
   - at least one probe for magnetic resonance imaging.

According to a particular embodiment, the nanovector according to the invention comprises:
- an external layer comprising a plurality of amphiphilic molecules, said amphiphilic molecules comprising at least one hydrophobic perfluorinated moiety linked to at least one hydrophilic moiety, and
- a core comprising:
   - at least one organometallic complex having a paramagnetic property that can be activated by oxidation, said organometallic complex being functionalized by at least one hydrophobic perfluorinated moiety; and
   - at least one fluorinated probe for ¹⁹F magnetic resonance imaging.

Advantageously and without wishing to be bound by the theory, it is proposed that the fluorinated amphiphilic molecules forms a fluorophilic space at the core of nanovectors, which can be used as a reservoir for the fluorinated organometallic complex as described here-above and the probe for ¹⁹F-MRI as defined above, more particularly the probe PERFECTA of formula (**IV**) as defined above, and also other fluorinated compounds such as fluorinated near infrared-responsive photosensitizer as described above.

Without wishing to be bound by the theory, the inventors are of the opinion that a fluorophilic space at the core of the nanovectors advantageously allows to further transport oxygen.

According to a particular embodiment, the nanovector according to the invention comprises:
- an external layer comprising a plurality of amphiphilic molecules comprising, notably consisting of, compounds of formula (**I**) as defined above or a mixture of compounds of formula (**I**) as defined above, in particular compounds of formula (**I**') as defined above and
- a core comprising:
   - at least one organometallic complex being a compound of formula (**II**) as defined above or a mixture of compounds of formula (**II**) as defined above, wherein R¹ and R² independently represent a hydrophobic perfluorinated moiety, in particular a compound of formula (**II**') as defined above; and
   - at least one probe for ¹⁹F magnetic resonance imaging.

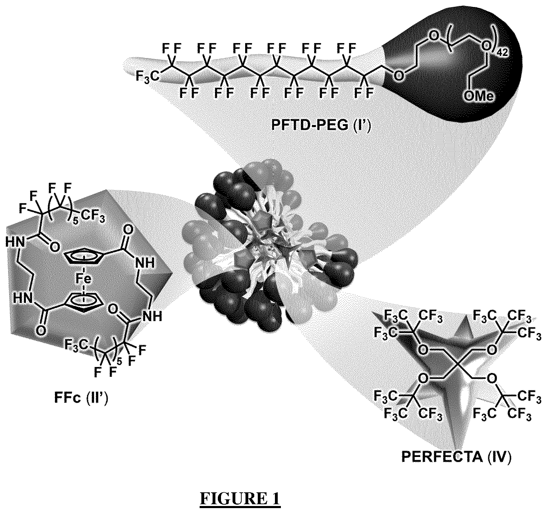

According to a particular embodiment, in particular as illustrated in **figure 1****,** the nanovector according to the invention comprises:
- an external layer comprising a plurality of amphiphilic molecules comprising, notably consisting of, compounds of formula (**I**) as defined above or a mixture of compounds of formula (**I**) as defined above, in particular compounds of formula (**I**') as defined above and
- a core comprising:
   - at least one organometallic complex being a compound of formula (**II**) as defined above or a mixture of compounds of formula (**II**) as defined above, wherein R¹ and R² independently represent a hydrophobic perfluorinated moiety, in particular a compound of formula (**II**') as defined above; and
   - at least one probe for ¹⁹F magnetic resonance imaging being a compound of formula (**IV**) as defined above.

According to a particular embodiment, the nanovector according to the invention comprises:
- an external layer comprising a plurality of amphiphilic molecules having a hydrophilic moiety comprising a polyethylene glycol (PEG), in particular comprising, notably consisting of, compounds of formula (**I**) as defined above or mixtures of compounds of formula (**I**) as defined above, more particularly compounds of formula (**I'**) as defined above and
- a core comprising:
   - at least one organometallic complex being a compound of formula (**II**) as defined above or a mixture of compounds of formula (**II**) as defined above, in particular wherein R¹ and R² independently represent a hydrophobic perfluorinated moiety, more particularly being of formula (**II**') as defined above; and
   - at least one probe for magnetic resonance imaging, in particular at least one fluorinated probe for ¹⁹F magnetic resonance imaging,
the probe for magnetic resonance imaging being present in an amount from 1% to 60% by weight, in particular from 10% to 50% by weight, more particularly from 20% to 50% by weight, notably from 30% to 50% by weight, relative to the total weight of the nanovector.

According to a particular embodiment, the nanovector according to the invention comprises:
- an external layer comprising a plurality of amphiphilic molecules comprising, notably consisting of, compounds of formula (**I**') as defined above and
- a core comprising:
   - at least one organometallic complex comprising at least, notably consisting of, the compound of formula (**II'**) as defined above; and
   - at least one compound of formula (**IV**) as defined above.

According to a particular embodiment, the nanovector according to the invention comprises:
- an external layer comprising a plurality of amphiphilic molecules comprising, notably consisting of, compounds of formula (**I**) as defined above or mixtures of compounds of formula (**I**) as defined above, in particular compounds of formula (**I'**) as defined above and
- a core comprising:
   - at least one organometallic complex being a compound of formula (**II**) as defined above or a mixture of compounds of formula (**II**) as defined above, wherein R¹ and R² independently represent a hydrophobic perfluorinated moiety, in particular being of formula (**II'**) as defined above; and
   - at least one probe for ¹⁹F magnetic resonance imaging, in particular being compound of formula (**IV**) as defined above,
the fluorinated probe for ¹⁹F magnetic resonance imaging being present in an amount from 1% to 60% by weight, in particular from 10% to 50% by weight, more particularly from 20% to 50% by weight, notably from 30% to 50% by weight, relative to the total weight of the nanovector.

According to a particular embodiment, the nanovector according to the invention comprises:
- an external layer comprising a plurality of amphiphilic molecules comprising, notably consisting of, compounds of formula (**I**) as defined above or mixtures of compounds of formula (**I**) as defined above, more particularly compounds of formula (**I**') as defined above and
- a core comprising:
   - at least one organometallic complex being a compound of formula (**II)** as defined above or a mixture of compounds of formula (**II**) as defined above, wherein R¹ and R² independently representing a hydrophobic perfluorinated moiety, more particularly being of formula (**II'**) as defined above; and
   - at least one probe for ¹⁹F magnetic resonance imaging, in particular being compound of formula (**IV**) as defined above,

the organometallic complex being present in an amount from 1% to 50% by weight, in particular from 5% to 40 % by weight, and more particularly from 10% to 30% by weight, relative to the total weight of the nanovector, and
the fluorinated probe for ¹⁹F magnetic resonance imaging being present in an amount from 1% to 60% by weight, in particular from 10% to 50% by weight, more particularly from 20% to 50% by weight, notably from 30% to 50% by weight, relative to the total weight of the nanovector.

According to a particular embodiment, the nanovector according to the invention comprises:
- an external layer comprising a plurality of amphiphilic molecules, said amphiphilic molecules comprising at least one hydrophobic moiety linked to at least one hydrophilic moiety, and
- a core comprising:
   - at least one organometallic complex having a paramagnetic property that can be activated by oxidation, said organometallic complex being functionalized by at least one hydrophobic moiety;
   - at least one near infrared-responsive photosensitizer suitable to produce oxidizing species when exposed to near infrared light and being functionalized by at least one hydrophobic moiety; and
   - at least one probe for magnetic resonance imaging.

According to this embodiment, the organometallic complex, the near infrared-responsive photosensitizer and the probe for magnetic resonance imaging are advantageously co-encapsulated by the external layer, in particular by a shell comprising, notably consisting of, amphiphilic molecules as described above.

Advantageously, the encapsulation of the organometallic complex, the near infrared-responsive photosensitizer and the probe for magnetic resonance imaging in a nanovector allows to enhance their solubility, in particular in aqueous solvent, more particularly in water.

Without wishing to be bound by the theory, it is proposed that, under near infrared illumination at the absorption band or one of the absorption bands of the photosensitizer, near infrared-responsive photosensitizer as defined above, in particular of formula (**III**) as defined above, more particularly of formula (**III**') as defined above, is excited and produces oxidizing species, in particular singlet oxygen (¹O₂), which, in turn, oxidizes the organometallic complex, in particular of formula (**II**) as defined above, more particularly of formula (**II**') as defined above, and thus extinguishes the MRI signal of the probe, in particular the probe for ¹⁹F-MRI, more particularly of formula (**IV**).

According to a particular embodiment, the nanovector according to the invention comprises:
- an external layer comprising a plurality of amphiphilic molecules, said amphiphilic molecules comprising at least one hydrophobic perfluorinated moiety linked to at least one hydrophilic moiety, and
- a core comprising:
   - at least one organometallic complex having a paramagnetic property that can be activated by oxidation, said organometallic complex being functionalized by at least one hydrophobic perfluorinated moiety;
   - at least one near infrared-responsive photosensitizer suitable to produce oxidizing species when exposed to near infrared light and being functionalized by at least one hydrophobic perfluorinated moiety; and
   - at least one fluorinated probe for ¹⁹F magnetic resonance imaging.

According to a particular embodiment, the nanovector according to the invention comprises:
- an external layer comprising a plurality of amphiphilic molecules comprising, notably consisting of, compounds of formula (**I**) as defined above or mixtures of compounds of formula (**I**) as defined above, in particular compounds of formula (**I'**) as defined above; and
- a core comprising:
   - at least one organometallic complex being a compound of formula (**II**) as defined above or a mixture of compounds of formula (**II**) as defined above, wherein R¹ and R² independently represent a hydrophobic perfluorinated moiety, in particular a compound of formula (**II**') as defined above;
   - at least one near infrared-responsive photosensitizer comprising at least a compound of formula (**III**) as defined above or a mixture of compounds of formula (**III**) as defined above, wherein B comprises a hydrophobic perfluorinated moiety, in particular a compound of formula (**III**') as defined above; and
   - at least one probe for ¹⁹F magnetic resonance imaging.

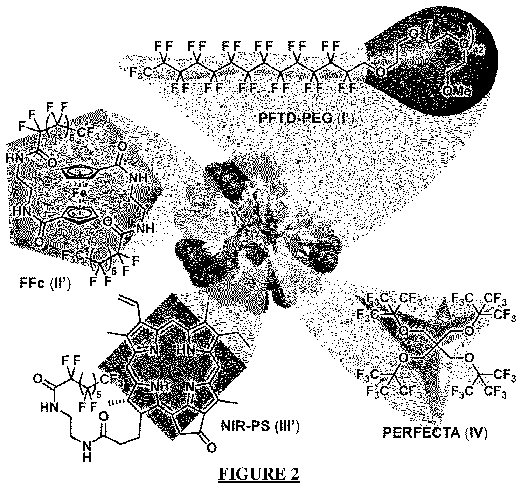

According to a particular embodiment, in particular as illustrated in **figure 2****,** the nanovector according to the invention comprises:
- an external layer comprising a plurality of amphiphilic molecules comprising, notably consisting of, compounds of formula (**I**) as defined above or mixtures of compounds of formula (**I**) as defined above, in particular compounds of formula (**I'**) as defined above; and

- a core comprising:
   - at least one organometallic complex being a compound of formula (**II**) as defined above or a mixture of compounds of formula (**II**) as defined above, wherein R¹ and R² independently represent a hydrophobic perfluorinated moiety, in particular a compound of formula (**II**') as defined above;
   - at least one near infrared-responsive photosensitizer comprising at least a compound of formula (**III**) as defined above or a mixture of compounds of formula (**III**) as defined above, wherein B comprises a hydrophobic perfluorinated moiety, in particular compound of formula (**III**') as defined above; and
   - at least one probe for ¹⁹F magnetic resonance imaging being a compound of formula (**IV**) as defined above.

According to a particular embodiment, the nanovector according to the invention comprises:
- an external layer comprising a plurality of amphiphilic molecules comprising, notably consisting of, compounds of formula (**I**) as defined above or mixtures of compounds of formula (**I**) as defined above and
- a core comprising:
   - at least one organometallic complex comprising at least, notably consisting of, the compound of formula (**II'**) as defined above;
   - at least one near infrared-responsive photosensitizer comprising at least, notably consisting of, the compound of formula (**III**') as defined above; and
   - at least one compound of formula (**IV**) as defined above.

According to a particular embodiment, the nanovector according to the invention comprises:
- an external layer comprising a plurality of amphiphilic molecules comprising at least, notably consisting of, compounds of formula (**I**') as defined above and
- a core comprising:
   - at least one organometallic complex comprising at least, notably consisting of, the compound of formula (**II'**) as defined above;
   - at least one near infrared-responsive photosensitizer comprising at least, notably consisting of, the compound of formula (**III**') as defined above; and
   - at least one compound of formula (**IV**) as defined above.

According to a particular embodiment, the nanovector according to the invention comprises:
- an external layer comprising a plurality of amphiphilic molecules comprising, notably consisting of, compounds of formula (**I**) as defined above or mixtures of compounds of formula (I) as defined above, in particular compounds of formula (I') as defined above and
- a core comprising:
   - at least one organometallic complex comprising at least the compound of formula (**II**) as defined above or a mixture of compounds of formula (**II**) as defined above, wherein R¹ and R² independently represent a hydrophobic perfluorinated moiety, in particular compound of formula (II**'**) as defined above;
   - at least one near infrared-responsive photosensitizer comprising at least the compound of formula (**III**) as defined above or a mixture of compounds of formula (**III**) as defined above, wherein B comprises a hydrophobic perfluorinated moiety, in particular compound of formula (**III**') as defined above; and
   - at least one probe for ¹⁹F magnetic resonance imaging, in particular being a compound of formula (**IV**) as defined above,

the organometallic complex being present in an amount from 1% to 50% by weight, in particular from 5% to 40 %, and more particularly from 10% to 30% by weight, relative to the total weight of the nanovector,
the near infrared-responsive photosensitizer being present in an amount from 0,1% to 5% by weight, in particular from 0,3% to 2% by weight, relative to the total weight of the nanovector, and
the fluorinated probe for ¹⁹F magnetic resonance imaging being present in an amount from 1% to 60% by weight, in particular from 10% to 50% by weight, more particularly from 20% to 50% by weight, notably from 30% to 50% by weight, relative to the total weight of the nanovector.

According to a particular embodiment, the nanovector further comprises at least one targeting agent.

According to a particular embodiment, the targeting agent is chosen from a molecular ligand, in particular biotin, folic acid or derivatives thereof, biomolecules, in particular antibody fragments, aptamers or derivatives thereof, or mixtures thereof.

According to a particular embodiment, the targeting agent is chosen from compounds suitable to provide affinity to specific cellular subpopulations, in particular cancer cells.

According to a particular embodiment, the targeting agent is on the surface of the nanovector.

### Composition

According to a **second main embodiment,** the invention relates to a composition, comprising water and a plurality of nanovectors as defined above.

According to a particular embodiment, herein is further provided a composition as described above, wherein the amphiphilic molecules are present at a concentration superior or equal to the critical micelle concentration of said amphiphilic molecules.

According to a particular embodiment, the composition comprises at least 0,1 g/L of amphiphilic molecules, in particular from 5 g/L to 50 g/L of amphiphilic molecules, more particularly from 10 g/L to 50 g/L of amphiphilic molecules.

Preferably, the composition is biocompatible.

According to a **third main embodiment,** the invention relates to a pharmaceutical or diagnostic composition, comprising a plurality of nanovectors as defined above and at least one pharmaceutically acceptable excipient.

In particular, the amphiphilic molecules are present at a concentration superior or equal to the critical micelle concentration of said amphiphilic molecules. In particular, the composition comprises at least 0,1 g/L of amphiphilic molecules, in particular from 5 g/L to 50 g/L of amphiphilic molecules, more particularly from 10 g/L to 50 g/L of amphiphilic molecules.

In particular, the composition according to the invention can further include one or more additives such as diluents, excipients, stabilizers and preservatives. Such additives are well known to those skilled in the art.

The aforementioned excipients may be selected according to the dosage form and the desired mode of administration.

Compositions of this invention may be administered in any manner, including, but not limited to, orally, parenterally, sublingually, transdermally, vaginally, rectally, transmucosally, topically, intranasally via inhalation, via buccal or intranasal administration, or combinations thereof. Parenteral administration includes, but is not limited to, intravenous, intra-arterial, intra-peritoneal, subcutaneous, intramuscular, intra-thecal, and intra-articular. The compositions of this invention may also be administered in the form of an implant, which allows slow release of the compositions as well as a slow controlled i.v. infusion.

For example, a nanovector according to the present invention can be present in any pharmaceutical form which is suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions.

### Method for preparing nanovectors

According to a **fourth main embodiment,** the invention relates to a method for preparing nanovectors as defined above, comprising at least the steps of:
a) providing an aqueous composition comprising:
   - a plurality of amphiphilic molecules, said amphiphilic molecules comprising at least one hydrophobic moiety linked to at least one hydrophilic moiety, said amphiphilic molecules being in particular as defined above;
   - at least one organometallic complex having a paramagnetic property that can be activated by oxidation, said organometallic complex being functionalized by at least one hydrophobic moiety, said organometallic complex being in particular as defined above;
   - optionally at least one near infrared-responsive photosensitizer suitable to produce oxidizing species when exposed to near infrared light and being functionalized by at least one hydrophobic moiety, said near infrared-responsive photosensitizer being in particular as defined above; and
   - optionally at least one probe for magnetic resonance imaging, in particular at least one fluorinated probe for ¹⁹F magnetic resonance imaging, said probe being in particular as defined above; and
b) mixing, in particular ultra-sonicating, the aqueous composition of step a);
   thereby preparing the nanovectors.

In particular, at step a):
- the organometallic complex is photo-responsive; and/or
- the aqueous composition comprises at least one near infrared-responsive photosensitizer suitable to produce oxidizing species when exposed to near infrared light and being functionalized by at least one hydrophobic moiety.

In particular, the aqueous composition at step a) may comprise:
- a plurality of amphiphilic molecules, said plurality of amphiphilic molecules having a hydrophilic moiety comprising a polyethylene glycol (PEG), in particular comprising, notably consisting of, compounds of formula (**I**) as defined above or mixtures of compounds of formula (**I**) as defined above, more particularly compounds of formula (**I**') as defined above; and
- at least one organometallic complex comprising at least one ferrocenyl group, in particular being a compound of formula (**II**) as defined above or a mixture of compounds of formula (**II**) as defined above, more particularly of formula (**II**') as defined above.

According to a particular embodiment, the method for preparing nanovectors according to the invention, comprises at least the steps of:
a) providing an aqueous composition comprising:
   - a plurality of amphiphilic molecules, said amphiphilic molecules comprising at least one hydrophobic moiety linked to at least one hydrophilic moiety, in particular as defined above;
   - at least one organometallic complex having a paramagnetic property that can be activated by oxidation, said organometallic complex being functionalized by at least one hydrophobic moiety, in particular as defined above;
   - at least one near infrared-responsive photosensitizer suitable to produce oxidizing species when exposed to near infrared light and being functionalized by at least one hydrophobic moiety, in particular as defined above; and
   - optionally at least one probe for magnetic resonance imaging, in particular at least one fluorinated probe for ¹⁹F magnetic resonance imaging, more particularly as defined above; and
b) mixing, in particular ultra-sonicating, the aqueous composition of step a);
   thereby preparing the nanovectors.

In particular, the aqueous composition at step a) comprises:
- a plurality of amphiphilic molecules, said plurality of amphiphilic molecules having a hydrophilic moiety comprising a polyethylene glycol (PEG), in particular comprising, notably consisting of, compounds of formula (**I**) as defined above or mixtures of compounds of formula (**I**) as defined above, more particularly compounds of formula (**I**') as defined above;
- at least one organometallic complex comprising at least one ferrocenyl group, in particular being a compound of formula (**II**) as defined above or a mixture of compounds of formula (**II**) as defined above, more particularly of formula (**II**') as defined above; and
- at least one near infrared-responsive photosensitizer comprising at least one group chosen from a pyropheophorbide-a group or a derivative thereof, in particular being a compound of formula (**III**) as defined above or a mixture of compounds of formula (**III**) as defined above, more particularly of formula (**III'**) as defined above.

According to a particular embodiment, the method for preparing nanovectors according to the invention, comprises at least the steps of:
a) providing an aqueous composition comprising:
   - a plurality of amphiphilic molecules, said amphiphilic molecules comprising at least one hydrophobic moiety linked to at least one hydrophilic moiety, in particular as defined above;
   - at least one organometallic complex having a paramagnetic property that can be activated by oxidation, said organometallic complex being functionalized by at least one hydrophobic moiety, in particular as defined above;
   - at least one probe for magnetic resonance imaging, in particular at least one fluorinated probe for ¹⁹F magnetic resonance imaging, more particularly as defined above; and
   - optionally at least one near infrared-responsive photosensitizer suitable to produce oxidizing species when exposed to near infrared light and being functionalized by at least one hydrophobic moiety, in particular as defined above; and
b) mixing, in particular ultra-sonicating, the aqueous composition of step a);
   thereby preparing the nanovectors.

In particular, the aqueous composition at step a) comprises:
- a plurality of amphiphilic molecules, said plurality of amphiphilic molecules comprising, notably consisting of, compounds of formula (**I**) as defined above or mixtures of compounds of formula (**I**) as defined above, more particularly compounds of formula (**I'**) as defined above;
- at least one organometallic complex being a compound of formula (**II**) as defined above, or a mixture of compounds of formula (**II**) as defined above, wherein R¹ and R² independently represent a hydrophobic perfluorinated moiety, in particular compound of formula (**II**') as defined above; and
- at least one probe for ¹⁹F magnetic resonance imaging, in particular being a compound of formula (**IV**).

According to a particular embodiment, the method for preparing nanovectors according to the invention, comprises at least the steps of:
a) providing an aqueous composition comprising:
   - a plurality of amphiphilic molecules, said amphiphilic molecules comprising at least one hydrophobic moiety linked to at least one hydrophilic moiety, in particular as defined above;
   - at least one organometallic complex having a paramagnetic property that can be activated by oxidation, said organometallic complex being functionalized by at least one hydrophobic moiety, in particular as defined above;
   - at least one near infrared-responsive photosensitizer suitable to produce oxidizing species when exposed to near infrared light and being functionalized by at least one hydrophobic moiety, in particular as defined above; and
   - at least one probe for magnetic resonance imaging, in particular at least one fluorinated probe for ¹⁹F magnetic resonance imaging, more particularly as defined above; and
b) mixing, in particular ultra-sonicating, the aqueous composition of step a);
   thereby preparing the nanovectors.

In particular, the aqueous composition at step a) comprises:
- a plurality of amphiphilic molecules, said plurality of amphiphilic molecules comprising, notably consisting of, compounds of formula (**I**) as defined above or mixtures of compounds of formula (**I**) as defined above, more particularly compounds of formula (**I'**) as defined above;
- at least one organometallic complex being a compound of formula (**II**) as defined above, or a mixture of compounds of formula (**II**) as defined above, wherein R¹ and R² independently represent a hydrophobic perfluorinated moiety, in particular compound of formula (**II**') as defined above;
- at least one near infrared-responsive photosensitizer comprising at least the compound of formula (**III**) as defined above or a mixture of compounds of formula (**III**) as defined above, wherein B comprises a hydrophobic perfluorinated moiety, in particular compound of formula (**III**') as defined above; and
- at least one probe for ¹⁹F magnetic resonance imaging, in particular being a compound of formula (**IV**).

Advantageously, at step a), the amphiphilic molecules, in particular of formula (**I**), more particularly of formula (**I**'), are present in the aqueous composition at a concentration superior or equal to the critical micelle concentration of said amphiphilic molecules, in particular the aqueous composition comprises at least 0,1 g/L of amphiphilic molecules, more particularly from 5 g/L to 50 g/L of amphiphilic molecules, even more particularly from 10 g/L to 50 g/L of amphiphilic molecules.

In particular, at step a), the aqueous composition may comprise from 0.5 g/L to 20 g/L of said organometallic complex, in particular of formula (**II**), more particularly of formula (**II**').

In particular, at step a), the aqueous composition may comprise from 0.05 g/L to 5 g/L of said near infrared-responsive photosensitizer, in particular of formula (**III**), more particularly of formula (**III**').

In particular, at step a), the aqueous composition may comprise from 1 g/L to 50 g/L of said probe, in particular of fluorinated probe for ¹⁹F magnetic resonance imaging, more particularly of formula (**IV**).

At step b), the aqueous composition may be mixed by a stirrer, for instance at high shear, or by ultrasound, in particular by ultrasound.

In particular, the aqueous solution is ultrasonicated, in particular for at least 5 min, more particularly at least 10 min, even more particularly at least 15 min, even more particularly from 15 min to 25 min.

According to a particular embodiment, the method for preparing nanovectors according to the invention may further comprise a step of c) of filtering the aqueous composition of step b), in particular through a membrane having a pore size lower or equal to 1 µm.

In particular, the nanovectors may be obtained in a dry form. For instance, the method for preparing nanovectors according to the invention may further comprise a step of lyophilization.

The nanovectors may also be obtained dispersed in an aqueous solution.

### Other embodiments

According to a **fifth main embodiment,** the invention relates to a nanovector according to the invention, in particular as defined above, a composition according to the invention, in particular as defined above, or a pharmaceutical or diagnostic composition according to the invention, in particular as defined above, for use in *in vivo* diagnostics. According to a **sixth main embodiment,** the invention relates to a nanovector according to the invention, in particular as defined above, a composition according to the invention, in particular as defined above, or a pharmaceutical or diagnostic composition according to the invention, in particular as defined above, for use in a method of magnetic resonance imaging, in particular ¹⁹F magnetic resonance imaging. The method of magnetic resonance imaging may be as defined here-below.

In particular, according to the fifth and sixth main embodiments, the nanovector further comprises at least one near infrared-responsive photosensitizer suitable to produce oxidizing species when exposed to near infrared light and being functionalized by at least one hydrophobic moiety, said near infrared-responsive photosensitizer being in particular as described above.

In particular, according to the fifth and sixth main embodiments, the nanovector further comprises at least one probe for magnetic resonance imaging, in particular at least one fluorinated probe for ¹⁹F magnetic resonance imaging, said probe for magnetic resonance imaging being in particular as described above.

Preferably, according to the fifth and sixth main embodiments, the nanovector further comprises:
- at least one near infrared-responsive photosensitizer suitable to produce oxidizing species when exposed to near infrared light and being functionalized by at least one hydrophobic moiety, said near infrared-responsive photosensitizer being in particular as described above; and
- at least one probe for magnetic resonance imaging, in particular at least one fluorinated probe for ¹⁹F magnetic resonance imaging, said probe for magnetic resonance imaging being in particular as described above.

Herein is further disclosed a diagnostic method comprising a step of administering the nanovector according to the invention to an individual/patient in need thereof. Preferably, said diagnostic method is an *in vivo* method. Alternatively, said diagnostic method may be an *ex vivo* method.

Here is further disclosed a use of a nanovector as defined above in a diagnostic method, in particular as defined above.

Here is further disclosed a use of a nanovector as defined above for the manufacture of a diagnostic composition.

Herein is further disclosed a method of magnetic resonance imaging comprising a step of a administering the nanovector according to the invention to an individual/patient in need thereof. The method of magnetic resonance imaging may as defined here-below.

Here is further disclosed a use of a nanovector as defined above in a method of magnetic resonance imaging, in particular as defined above.

Here is further disclosed a use of a nanovector as defined above for the manufacture of a composition for a method of magnetic resonance imaging, in particular as defined above.

In said methods, the nanovector may further comprise at least one near infrared-responsive photosensitizer as described above and/or at least one probe for magnetic resonance imaging as described above.

### Method of magnetic resonance imaging

According to a **seventh main embodiment,** the invention relates to a method of magnetic resonance imaging also called MRI, comprising at least the steps of:
i) exposing to light zone(s) of a patient previously administered with nanovector(s) according to the invention, in particular as defined above, in particular with a composition according to the invention or a pharmaceutical or diagnostic composition according to the invention, said zone(s) being not to be imaged by MRI; and
ii) imaging zone(s) of interest of said patient by MRI, the zone(s) of steps i) and ii) being distinct,
step ii) being consecutive or simultaneous with step i).

In particular, step ii) follows step i). In particular, step i) is performed 1 min to 15 min, in particular 1 min to 5 min, before step ii).

In particular, at step (i), the light is blue light and/or near infrared light, more particularly at a wavelength comprised between 300 nm and 1200 nm, even more particularly between 400 nm and 800 nm, for example between 400 and 700 nm.

In particular, the core of said nanovector(s) further comprises at least one near infrared-responsive photosensitizer suitable to produce oxidizing species when exposed to near infrared light and being functionalized by at least one hydrophobic moiety, in particular as defined above.

In particular, at step i):
- the organometallic complex of said nanovector(s) is photo-responsive and said light is at a wavelength corresponding to the absorption band of the photo-responsive organometallic complex or one of the absorption bands of the photo-responsive organometallic complex; and/or
- the core of said nanovector(s) further comprises at least one near infrared-responsive photosensitizer suitable to produce oxidizing species when exposed to near infrared light and being functionalized by at least one hydrophobic moiety, in particular as defined above, and the light is at a wavelength corresponding to the absorption band of the near infrared-responsive photosensitizer or one of the absorption bands of the near infrared-responsive photosensitizer.

In particular, the core of said nanovector(s) further comprises at least one probe for magnetic resonance imaging, in particular as defined above.

In particular, MRI is ¹H-MRI, ¹⁹F-MRI, ¹³C-MRI, ²H-MRI or ³¹P-MRI, more particularly ¹⁹F-MRI.

In particular, MRI is ¹⁹F-MRI, ¹³C-MRI, ²H-MRI or ³¹P-MRI, and the core of said nanovector(s) further comprises at least one probe for magnetic resonance imaging chosen among probes for ¹⁹F-MRI, ¹³C-MRI, ²H-MRI or ³¹P-MRI, in particular as defined above. The person skilled in the art may select the probe for MRI suitable for the type of MRI used.

According to a particular embodiment, the core of the nanovector(s) further comprises at least one fluorinated probe for ¹⁹F magnetic resonance imaging, in particular as described above, and MRI is ¹⁹F-MRI.

Magnetic resonance imaging is well known to those skilled in the art and is described notably in "MRI Basic Principles and Applications" Brian M. Dale, Mark A. Brown, Richard C. Semelka, 2015, John Wiley & Sons. In particular, at step ii), MRI may be performed with a 7 T Biospec preclinical scanner (Bruker).

According to a particular embodiment, the method of MRI according to the invention is a method of ¹H-MRI, ¹³C-MRI or ²H-MRI, comprising at least the steps of:
i) exposing to light zone(s) of a patient previously administered with nanovector(s), in particular as defined above, comprising:
   - an external layer comprising a plurality of amphiphilic molecules, said amphiphilic molecules comprising at least one hydrophobic moiety linked to at least one hydrophilic moiety, in particular as described above, and
   - a core comprising at least one photo-responsive organometallic complex having a paramagnetic property that can be activated by oxidation, said organometallic complex being functionalized by at least one hydrophobic moiety, in particular as described above,

   said zone(s) being not to be imaged by MRI, and
   said light being at a wavelength corresponding to the absorption band or one of the absorption bands of the photo-responsive organometallic complex, in particular at a wavelength comprise between 300 nm and 800 nm, more particularly between 380 nm and 520 nm, even more particularly between 400 nm and 500 nm, for instance at 450 nm,
ii) imaging zone(s) of interest of said patient by ¹H-MRI, ¹³C-MRI or ²H-MRI, the zone(s) of steps i) and ii) being distinct,
   step ii) being consecutive or simultaneous with step i).

In particular, exposing to light at a wavelength corresponding to the absorption band or one of the absorption bands of the organometallic complex induces oxidation of the organometallic complex.

According to this particular embodiment, at step i), the nanovector(s) preferably comprises:
- an external layer comprising a plurality of amphiphilic molecules having a hydrophilic moiety comprising a polyethylene glycol (PEG), in particular comprising, notably consisting of, compounds of formula (I) as defined above or mixtures of compounds of formula (**I**) as defined above, more particularly compounds of formula (**I**') as defined above, and
- a core comprising at least one organometallic complex comprising at least one ferrocenyl group, in particular being a compound of formula (**II**) as defined above or a mixture of compounds of formula (**II**) as defined above, more particularly of formula (**II**') as defined above.

According to another particular embodiment of the method of MRI according to the invention, said light at step i) is near infrared light, in particular at a wavelength between 600 nm and 1200 nm, more particularly between 600 nm and 800 nm, even more particularly between 620 nm and 700 nm, and nanovector(s) comprise(s):
- an external layer comprising a plurality of amphiphilic molecules, said amphiphilic molecules comprising at least one hydrophobic moiety linked to at least one hydrophilic moiety, in particular as described above, and
- a core comprising:
   - at least one organometallic complex having a paramagnetic property that can be activated by oxidation, said organometallic complex being functionalized by at least one hydrophobic moiety, in particular as described above; and
   - at least one near infrared-responsive photosensitizer suitable to produce oxidizing species when exposed to near infrared light and being functionalized by at least one hydrophobic moiety, in particular as described above.

Advantageously, said light is at a wavelength corresponding to the absorption band or one of the absorption bands of the near infrared-responsive photosensitizer.

The presence of the near infrared-responsive photosensitizer is particularly advantageous for imaging deep soft tissues, as it allows to modulate the MRI signal at near infrared light.

According to this particular embodiment, at step i), the nanovector(s) may comprise:
- an external layer comprising a plurality of amphiphilic molecules comprising, notably consisting of, compounds of formula (**I**) as defined above or mixtures of compounds of formula (**I**) as defined above, in particular compounds of formula (**I'**) as defined above and
- a core comprising:
   - at least one organometallic complex being a compound of formula (**II**) as defined above or a mixture of compounds of formula (**II**) as defined above, in particular a compound of formula (**II**') as defined above; and
   - at least one near infrared-responsive photosensitizer being compound of formula (**III**) as defined above or a mixture of compounds of formula (**III**) as defined above, in particular a compound of formula (**III**') as defined above.

According to another particular embodiment, the method according to the invention is a method of ¹H-MRI, ¹³C-MRI or ²H-MRI, comprising at least the steps of:
i) exposing to light zone(s) of a patient previously administered with nanovector(s), in particular as defined above, comprising:
   - an external layer comprising a plurality of amphiphilic molecules, said amphiphilic molecules comprising at least one hydrophobic moiety linked to at least one hydrophilic moiety, in particular as described above, and
   - a core comprising:
      - at least one organometallic complex having a paramagnetic property that can be activated by oxidation, said organometallic complex being functionalized by at least one hydrophobic moiety, in particular as described above; and
      - at least one near infrared-responsive photosensitizer suitable to produce oxidizing species when exposed to near infrared light and being functionalized by at least one hydrophobic moiety, in particular as described above,

   said zone(s) being not to be imaged by MRI, and
   said light being at a wavelength corresponding to the absorption band or one of the absorption bands of the near infrared-responsive photosensitizer, in particular at a wavelength comprised between 600 nm and 1200 nm, more particularly between 600 nm and 800 nm, even more particularly between 620 nm and 700 nm, for instance at 650 nm,
ii) imaging zone(s) of interest of said patient by ¹H-MRI, ¹³C-MRI or ²H-MRI, the zone(s) of steps i) and ii) being distinct,
   step ii) being consecutive or simultaneous with step i).

In particular, exposing to light at a wavelength corresponding to the absorption band or one of the absorption bands of the near infrared-responsive photosensitizer induces the production of oxidizing species from the near infrared-responsive photosensitizer.

According to this particular embodiment, at step i), the nanovector(s) preferably comprise(s):
- an external layer comprising a plurality of amphiphilic molecules having a hydrophilic moiety comprising a polyethylene glycol (PEG), in particular comprising, notably consisting of, compounds of formula (**I**) as defined above or mixtures of compounds of formula (**I**) as defined above, more particularly compounds of formula (**I**') as defined above, and
- a core comprising:
   - at least one organometallic complex comprising at least one ferrocenyl group, in particular being a compound of formula (**II**) as defined above or a mixture of compounds of formula (**II**) as defined above, more particularly of formula (**II**') as defined above; and
   - at least one near infrared-responsive photosensitizer as described above, comprising at least a group chosen from a pyropheophorbide-a group or a derivative thereof, in particular being of a compound of formula (**III**) as defined above or a mixture of compounds of formula (**III**) as defined above, more particularly of formula (**III**') as defined above.

According to a particular embodiment, the invention further relates to a method of MRI chosen among ¹⁹F-MRI, ¹³C-MRI, ²H-MRI and ³¹P-MRI, more particularly ¹⁹F-MRI, comprising at least the steps of:
i) exposing to light zone(s) of a patient previously administered with nanovector(s), in particular as defined above, comprising:
   - an external layer comprising a plurality of amphiphilic molecules, said amphiphilic molecules comprising at least one hydrophobic moiety linked to at least one hydrophilic moiety, in particular as described above, and
   - a core comprising:
      - at least one photo-responsive organometallic complex having a paramagnetic property that can be activated by oxidation, said organometallic complex being functionalized by at least one hydrophobic moiety, in particular as described above; and
      - at least one probe for ¹⁹F-MRI, ¹³C-MRI, ²H-MRI and ³¹P-MRI, in particular at least one fluorinated probe for ¹⁹F magnetic resonance imaging, in particular as described above,

   said zone(s) being not to be imaged by MRI, and
   said light being at a wavelength corresponding to the absorption band or one of the absorption bands of the photo-responsive organometallic complex, in particular at a wavelength comprise between 300 nm and 800 nm, more particularly between 380 nm and 520 nm, even more particularly between 400 nm and 500 nm, for instance at 450 nm,
ii) imaging zone(s) of interest of said patient by ¹⁹F-MRI, ¹³C-MRI, ²H-MRI or ³¹P-MRI, more particularly ¹⁹F-MRI, the zone(s) of steps i) and ii) being distinct,
   step ii) being consecutive or simultaneous with step i).

According to this particular embodiment, at step i), the nanovector(s) may comprise:
- an external layer comprising a plurality of amphiphilic molecules having a hydrophilic moiety comprising a polyethylene glycol (PEG), in particular comprising, notably consisting of, compounds of formula (**I**) as defined above or mixtures of compounds of formula (**I**) as defined above, more particularly compounds of formula (**I**') as defined above and
- a core comprising:
   - at least one organometallic complex being a compound of formula (**II**) as defined above or a mixture of compounds of formula (**II**) as defined above, in particular being of formula (**II**) as defined above wherein R¹ and R² independently representing a hydrophobic perfluorinated moiety, more particularly being of formula (**II**') as defined above; and
   - at least one probe for magnetic resonance imaging, in particular at least one fluorinated probe for ¹⁹F magnetic resonance imaging.

According to a particular embodiment, the invention further relates to a method of ¹⁹F-MRI, comprising at least the steps consisting of:
i) exposing to light, in particular blue light and/or near infrared light, more particularly at a wavelength comprised between 400 nm and 1200 nm, even more particularly between 400 nm and 800 nm, for example between 400 nm and 700 nm, zone(s) of a patient previously administered with nanovector(s) as defined above, comprising:
   - an external layer comprising a plurality of amphiphilic molecules, said amphiphilic molecules comprising at least one hydrophobic perfluorinated moiety linked to at least one hydrophilic moiety, in particular as described above, and
   - a core comprising:
      - at least one organometallic complex having a paramagnetic property that can be activated by oxidation, said organometallic complex being functionalized
      by at least one hydrophobic perfluorinated moiety, in particular as described above; and
   - at least one fluorinated probe for ¹⁹F-MRI, in particular as described above,
   said zone(s) being not to be imaged by ¹⁹F-MRI; and
ii) imaging zone(s) of interest of said patient by ¹⁹F-MRI, the zone(s) of steps i) and ii) being distinct,
   step ii) being consecutive or simultaneous with step i).

According to this particular embodiment, the nanovector(s) at step i) may comprise:
- an external layer comprising a plurality of amphiphilic molecules comprising, notably consisting of, compounds of formula **(I)** as defined above or a mixture of compounds of formula **(I)** as defined above, in particular compounds of formula **(I')** as defined above and
- a core comprising:
   - at least one organometallic complex being a compound of formula **(II)** as defined above or a mixture of compounds of formula **(II)** as defined above, wherein R¹ and R² independently represent a hydrophobic perfluorinated moiety, in particular a compound of formula **(II')** as defined above; and
- at least one probe for ¹⁹F magnetic resonance imaging, in particular being a compound of formula **(IV)** as defined above.

According to a particular embodiment, the invention further relates to a method of MRI chosen among ¹⁹F-MRI, ¹³C-MRI, ²H-MRI and ³¹P-MRI, more particularly ¹⁹F-MRI, comprising at least the steps of:
i) exposing to light zone(s) of a patient previously administered with nanovector(s), in particular as described above, comprising:
   - an external layer comprising a plurality of amphiphilic molecules, said amphiphilic molecules comprising at least one hydrophobic moiety linked to at least one hydrophilic moiety, and
   - a core comprising:
      - at least one organometallic complex having a paramagnetic property that can be activated by oxidation, said organometallic complex being functionalized by at least one hydrophobic moiety, in particular as described above;
      - at least one near infrared-responsive photosensitizer suitable to produce oxidizing species when exposed to near infrared light and being functionalized by at least one hydrophobic moiety, in particular as described above, and
      - at least one probe for ¹⁹F-MRI, ¹³C-MRI, ²H-MRI and ³¹P-MRI, in particular at least one fluorinated probe for ¹⁹F magnetic resonance imaging, in particular as described above,

   said zone(s) being not to be imaged by MRI,
   said light being at a wavelength corresponding to the absorption band or one of the absorption bands of the near infrared-responsive photosensitizer, in particular at a wavelength comprised between 600 nm and 1200 nm, more particularly between 600 nm and 800 nm, even more particularly between 620 nm and 700 nm, for instance at 650 nm; and
ii) imaging zone(s) of interest of said patient by ¹⁹F-MRI, ¹³C-MRI, ²H-MRI or ³¹P-MRI, more particularly ¹⁹F-MRI, the zone(s) of steps i) and ii) being distinct,
   step ii) being consecutive or simultaneous with step i).

According to a particular embodiment, the invention further relates to a method of ¹⁹F-MRI, comprising at least the steps of:
i) exposing to near infrared light, in particular at a wavelength between 600 nm and 1200 nm, more particularly between 600 nm and 800 nm, even more particularly between 620 nm and 700 nm, for instance at 650 nm, zone(s) of a patient previously administered with nanovector(s), in particular as described above, comprising:
   - an external layer comprising a plurality of amphiphilic molecules, said amphiphilic molecules comprising at least one hydrophobic perfluorinated moiety linked to at least one hydrophilic moiety, and
   - a core comprising:
      - at least one organometallic complex having a paramagnetic property that can be activated by oxidation, said organometallic complex being functionalized by at least one hydrophobic perfluorinated moiety;
      - at least one near infrared-responsive photosensitizer suitable to produce oxidizing species when exposed to near infrared light and being functionalized by at least one hydrophobic perfluorinated moiety; and
      - at least one fluorinated probe for ¹⁹F magnetic resonance imaging, said zone(s) being not to be imaged by ¹⁹F-MRI; and
ii) imaging zone(s) of interest of said patient by ¹⁹F-MRI, the zone(s) of steps i) and ii) being distinct,
   step ii) being consecutive or simultaneous with step i).

According to this particular embodiment, the nanovector(s) at step i) may comprise:
- an external layer comprising a plurality of amphiphilic molecules comprising, notably consisting of, compounds of formula **(I)** as defined above or mixtures of compounds of formula **(I)** as defined above, in particular compounds of formula **(I')** as defined above and
- a core comprising:
   - at least one organometallic complex being a compound of formula **(II)** as defined above or a mixture of compounds of formula **(II)** as defined above, wherein R¹ and R² independently represent a hydrophobic perfluorinated moiety, in particular a compound of formula **(II')** as defined above;
   - at least one near infrared-responsive photosensitizer comprising at least a compound of formula **(III)** as defined above or a mixture of compounds of formula (**III**) as defined above, wherein B comprises a hydrophobic perfluorinated moiety, in particular compound of formula **(III')** as defined above; and
   - at least one probe for ¹⁹F magnetic resonance imaging, in particular being compound of formula **(IV)** as defined above.

According to a particular embodiment, the invention further relates to a method of MRI chosen among ¹H-MRI and ¹⁹F-MRI comprising at least the steps of:
i) exposing to light zone(s) of a patient previously administered with nanovector(s), in particular as described above, comprising:
   - an external layer comprising a plurality of amphiphilic molecules, said amphiphilic molecules comprising at least one hydrophobic moiety linked to at least one hydrophilic moiety, in particular as defined above, and
   - a core comprising:
      - at least one photo-responsive organometallic complex having a paramagnetic property that can be activated by oxidation, said organometallic complex being functionalized by at least one hydrophobic moiety, in particular as described above;
      - optionally at least one near infrared-responsive photosensitizer suitable to produce oxidizing species when exposed to near infrared light and being functionalized by at least one hydrophobic moiety, in particular as described above, and
      - at least one fluorinated probe for ¹⁹F magnetic resonance imaging, in particular as described above,

   said zone(s) being not to be imaged by MRI,
   said light being at a wavelength corresponding to the absorption band or one of the absorption bands of the organometallic complex or, when the near infrared-responsive photosensitizer is present, corresponding to the absorption band or one of the absorption bands of the near infrared-responsive photosensitizer; and
ii) imaging zone(s) of interest of said patient by ¹H-MRI or ¹⁹F-MRI, the zone(s) of steps i) and ii) being distinct,
   step ii) being consecutive or simultaneous with step i).

### Kit for magnetic resonance imaging

According to an **eighth main embodiment,** the invention relates to a kit for magnetic resonance imaging, in particular for ¹H-MRI, ¹⁹F-MRI, ¹³C-MRI, ²H-MRI or ³¹P-MRI, more particularly for ¹⁹F-MRI, comprising:
- a plurality of amphiphilic molecules, said plurality of amphiphilic molecules comprising at least one hydrophobic moiety linked to at least one hydrophilic moiety, said amphiphilic molecules being in particular as describe above;
- at least one organometallic complex having a paramagnetic property that can be activated by oxidation, said organometallic complex being functionalized by at least one hydrophobic moiety, said organometallic complex being in particular as described above;
- optionally at least one near infrared-responsive photosensitizer suitable to produce oxidizing species when exposed to near infrared light and being functionalized by at least one hydrophobic moiety, said near infrared-responsive photosensitizer being in particular as described above; and
- optionally at least one probe for magnetic resonance imaging, in particular as described above.

In particular, the kit may be for preparing the nanovectors according to the invention.

In particular, the kit may comprise:
- a photo-responsive organometallic complex; and/or
- at least one near infrared-responsive photosensitizer suitable to produce oxidizing species when exposed to near infrared light and being functionalized by at least one hydrophobic moiety.

In particular, the kit according to the invention may comprise:
- a plurality of amphiphilic molecules, said plurality of amphiphilic molecules having a hydrophilic moiety comprising a polyethylene glycol (PEG), in particular comprising, notably consisting of, compounds of formula (I) as defined above or mixtures of compounds of formula **(I)** as defined above, more particularly compounds of formula **(I')** as defined above; and
- at least one organometallic complex comprising at least one ferrocenyl group, in particular being a compound of formula **(II)** as defined above or a mixture of compounds of formula **(II)** as defined above, more particularly of formula **(II')** as defined above.

According to a particular embodiment, the kit according to the invention may comprise:
- a plurality of amphiphilic molecules, said plurality of amphiphilic molecules comprising at least one hydrophobic moiety linked to at least one hydrophilic moiety, in particular as defined above;
- at least one organometallic complex having a paramagnetic property that can be activated by oxidation, said organometallic complex being functionalized by at least one hydrophobic moiety, in particular as defined above;
- at least one near infrared-responsive photosensitizer suitable to produce oxidizing species when exposed to near infrared light and being functionalized by at least one hydrophobic moiety, in particular as defined above; and
- optionally at least one probe for magnetic resonance imaging, in particular as defined above.

In particular, the kit according to the invention comprises:
- a plurality of amphiphilic molecules, said plurality of amphiphilic molecules having a hydrophilic moiety comprising a polyethylene glycol (PEG), in particular comprising, notably consisting of, compounds of formula **(I)** as defined above or mixtures of compounds of formula **(I)** as defined above, more particularly compounds of formula **(I')** as defined above;
- at least one organometallic complex comprising at least one ferrocenyl group, in particular being a compound of formula **(II)** as defined above or a mixture of compounds of formula **(II)** as defined above, more particularly of formula (**II**') as defined above; and
- at least one near infrared-responsive photosensitizer comprising at least a group chosen from a pyropheophorbide-a group or a derivative thereof, in particular being of a compound of formula **(III)** as defined above or a mixture of compounds of formula **(III)** as defined above, more particularly of formula (**III**') as defined above.

According to a particular embodiment, the kit according to the invention comprises:
- a plurality of amphiphilic molecules, said amphiphilic molecules comprising at least one hydrophobic moiety linked to at least one hydrophilic moiety, in particular as defined above;
- at least one organometallic complex having a paramagnetic property that can be activated by oxidation, said organometallic complex being functionalized by at least one hydrophobic moiety, in particular as defined above;
- at least one probe for magnetic resonance imaging, in particular at least one fluorinated probe for ¹⁹F magnetic resonance imaging, more particularly as defined above; and
- optionally at least one near infrared-responsive photosensitizer suitable to produce oxidizing species when exposed to near infrared light and being functionalized by at least one hydrophobic moiety, in particular as defined above.

In particular, the kit according to the invention comprises:
- a plurality of amphiphilic molecules, said plurality of amphiphilic molecules comprising, notably consisting of, compounds of formula **(I)** as defined above or mixtures of compounds of formula **(I)** as defined above, more particularly compounds of formula **(I')** as defined above;
- at least one organometallic complex being a compound of formula **(II)** as defined above, or a mixture of compounds of formula **(II)** as defined above, wherein R¹ and R² independently representing a hydrophobic perfluorinated moiety, and mixtures thereof, in particular compound of formula **(II')** as defined above; and
- at least one probe for ¹⁹F magnetic resonance imaging, in particular being a compound of formula **(IV).**

According to a particular embodiment, the kit according to the invention comprises:
- a plurality of amphiphilic molecules, said amphiphilic molecules comprising at least one hydrophobic moiety linked to at least one hydrophilic moiety, in particular as defined above;
- at least one organometallic complex having a paramagnetic property that can be activated by oxidation, said organometallic complex being functionalized by at least one hydrophobic moiety, in particular as defined above;
- at least one near infrared-responsive photosensitizer suitable to produce oxidizing species when exposed to near infrared light and being functionalized by at least one hydrophobic moiety, in particular as defined above; and
- at least one probe for magnetic resonance imaging, in particular at least one fluorinated probe for ¹⁹F magnetic resonance imaging, more particularly as defined above.

In particular, the kit according to the invention comprises:
- a plurality of amphiphilic molecules, said plurality of amphiphilic molecules comprising, notably consisting of, compounds of formula **(I)** as defined above or mixtures of compounds of formula **(I)** as defined above, more particularly compounds of formula **(I')** as defined above;
- at least one organometallic complex being a compound of formula **(II)** as defined above, or a mixture of compounds of formula **(II)** as defined above, wherein R¹ and R² independently representing a hydrophobic perfluorinated moiety, and mixtures thereof, in particular compound of formula **(II')** as defined above;
- at least one near infrared-responsive comprising at least the compound of formula **(III)** as defined above or a mixture of compounds of formula **(III)** as defined above, wherein B comprises a hydrophobic perfluorinated moiety, in particular compound of formula **(III')** as defined above; and
- at least one probe for ¹⁹F magnetic resonance imaging, in particular being a compound of formula **(IV).**

According to a particular embodiment, the plurality of amphiphilic molecules is dispersed in solution, in particular in aqueous solution, more particularly in water.

In particular, the plurality of amphiphilic molecules forms micelles, in particular dispersed micelles, in solution, in particular in aqueous solution, more particularly in water.

According to a particular embodiment, the plurality of amphiphilic molecules is in dry form, in particular obtained by a step of lyophilization of a solution containing said plurality of amphiphilic molecules.

### DEFINITIONS

As used in this specification and the appended claims, the singular forms **"a", "an"** and **"the"** include plural referents unless the content clearly dictates otherwise.

As used in this specification and the appended claims, the term **"at least one",** may thus include one, or **"more than one".** Accordingly, the terms **"a plurality of"** or **"more than one"** may thus include **"two"** or **"two or more".**

The term **"comprise"** is to be interpreted as specifying the presence of the stated features, integers, steps or components, but not precluding the presence of one or more other features, integers, steps or components, or group thereof. Also, it may specify strictly the stated features, integers, steps or components, and therefore in such case it may be replaced with **"consist of".**

As used herein, the term **"nanovector"** refers to a carrier nanosystem, in particular to a nanoparticle-based carrier system, with a size inferior or equal to 300 nm, comprising a hydrophobic core surrounded by a hydrophilic shell or corona. This hydrophobic core can be loaded with hydrophobic molecules, such as pharmaceutically active agents or contrast agents, and is surrounded by the hydrophilic shell, said shell allowing nanovectors to transport said hydrophobic molecules, in particular towards target tissues. In particular, a **"nanovector"** is a **"micelle".** As used herein, the term "micelle" refers to the aggregate of amphiphilic molecules or **"surfactants"** and optionally of hydrophobic molecules such as pharmaceutically active agents or contrast agents, dispersed in a liquid. As use herein, micelles can thus be defined as colloidal dispersions from amphiphilic molecules with a hydrophobic tail and a hydrophilic head. Such micelles form in the liquid after reaching the corresponding critical micelle concentration (or CMC) of the amphiphile/surfactant(s). Hence, the term may encompass both micelles assembled from one type of amphiphile and micelles assembled from a plurality of distinct amphiphiles; such as those micelles assembled from two or more distinct amphiphiles. Micelles may be characterized from other types of aggregates by the organization of the amphiphilic molecules into one or more ordered layers, and in particular monolayers. Micelles may comprise hydrophobic molecules encapsulated in the layer(s) of amphiphilic molecules. Unless specified otherwise, the term may encompass micelles or nanovectors having all ranges of hydrodynamic size inferior to 300 nm, in particular those having a hydrodynamic diameter at or below 40 nm, such as at or below 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8 or 7 nm. According to the invention, a nanovector may also be called nanometric micellar carrier system.

As used herein, the term **"amphiphile"** refers to molecules and/or components (e.g., functional groups/moieties, polymers, and blocks of block polymers, etc.) of molecules having at least one hydrophilic moiety and at least one hydrophobic moiety, said hydrophilic and hydrophobic moieties being optionally linked by one or more linker regions. In particular, the term may refer to such molecules and/or components of such molecules, which are able to associate or co-associate in order to form nanovectors, in particular micelles, at or above a given CMC.

As used herein, the term **"hydrophilic"** refers to molecules and/or components (e.g., functional groups/moieties, polymers, and blocks of block polymers, etc.) of molecules having at least one hydrophilic group, and the term **"hydrophobic"** refers to molecules and/or components (e.g., functional groups/moieties, polymers, and blocks of block copolymers etc.) of molecules having at least one hydrophobic group.

Hydrophilic molecules or components thereof tend to have ionic and/or polar groups, and hydrophobic molecules or components thereof tend to have nonionic and/or nonpolar groups. Hydrophilic molecules or components thereof tend to participate in stabilizing interactions with an aqueous solution, including hydrogen bonding and dipole-dipole interactions. Examples of hydrophilic molecules or components may comprise PEG polymers.

Hydrophobic molecules or components tend not to participate in stabilizing interactions with an aqueous solution and, thus often cluster together in an aqueous solution to achieve a more stable thermodynamic state. Hydrophobic molecules or components tend to participate in stabilizing interactions with other hydrophobic molecules or components.

Hydrophobic moieties/groups may comprise, in particular consist of, a substituted or unsubstituted, saturated or unsaturated, aliphatic chain-containing moiety or a hydrophobic perfluorinated moiety, in particular a substituted or unsubstituted, saturated or unsaturated, aliphatic C₄-C₃₆ chain-containing moiety or a hydrophobic perfluorinated moiety, more particularly a saturated or unsaturated aliphatic C₄-C₃₆ chain-containing moiety or a hydrophobic perfluorinated moiety, even more particularly a (C₄-C₃₆) alkyl moiety or a hydrophobic perfluorinated moiety.

In particular, hydrophobic moieties/groups may comprise, in particular consist of, a substituted or unsubstituted, saturated or unsaturated, aliphatic C₆-C₃₆ chain-containing moiety or a hydrophobic perfluorinated moiety, in particular a substituted or unsubstituted, saturated or unsaturated, aliphatic C₁₀-C₃₂ chain-containing moiety or a hydrophobic perfluorinated moiety, more particularly a substituted or unsubstituted, saturated or unsaturated, aliphatic C₁₄-C₂₈ chain-containing moiety or a hydrophobic perfluorinated moiety, even more particularly a substituted or unsubstituted, saturated or unsaturated, aliphatic C₁₈-C₂₈ chain-containing moiety or a hydrophobic perfluorinated moiety.

In particular, hydrophobic moieties/groups may comprise, in particular consist of, a (C₆-C₃₆) alkyl moiety or a hydrophobic perfluorinated moiety, in particular a (C₁₀-C₃₂) alkyl moiety or a hydrophobic perfluorinated moiety, more particularly a (C₁₄-C₂₈) alkyl moiety or a hydrophobic perfluorinated moiety, even more particularly a (C₁₈-C₂₈) alkyl moiety or a hydrophobic perfluorinated moiety.

As used herein, the terms **"average droplet size"** or **"average hydrodynamic size"** or **"average hydrodynamic diameter"** are used interchangeably. The average droplet size of a particle or droplet or micelle can be determined by any dynamic light scattering (DLS) technique, for example a dynamic light scattering measurement by a Cordouan Vasco-Flex apparatus, equipped with a 450 nm laser, at ambient temperature (e.g. at 20°C or 25°C) or on a Malvern Zetasizer Nano ZS (He-Ne laser 633 nm).

As used herein, the term **"organometallic complex"** refers to a compound comprising at least one metal atom and at least one metal complexing moiety, which comprises in particular at least one or more electron-donor atom(s) capable of forming coordinate bond(s) with said metal atom(s).

As used herein, the term **"metallocene group"** refers to a compound consisting of two cyclopentadienyl anions of formula C₅H⁻₅ and abbreviated Cp, which are bounded to a metal center (M) in the oxidation state II, with the resulting general formula (C₅H₅)₂M. In particular, the metal atom is chosen from transition metals, in particular from iron, copper or manganese, more particularly form iron.

As used herein, a compound said to have **"paramagnetic property"** refers to a compound with unpaired electrons and which is weakly attracted by an externally applied magnetic field, and acquires, under the effect of such field, a magnetization oriented in the direction of the applied magnetic field.

As used herein, the term **"light"** refers to electromagnetic radiation that can propagate at a specific wavelength, also called monochromatic light, or at several wavelengths such as visible light, in other words comprises at least two monochromatic lights. In particular, the term "light" refers to electromagnetic radiation having a wavelength comprised between 300 nm and 1200 nm, more particularly between 400 nm and 800 nm.

As used herein, the term **"illumination"** refers to an exposition to light.

As used herein, the term **"photo-responsive"** refers to a compound which undergoes a chemical reaction by absorption of light, in particular at one or more specific wavelengths corresponding to the absorption band or one of the absorption bands of the photo-responsive compound. In particular, a photo-responsive compound is oxidized upon light excitation at one or more specific wavelengths.

As used herein, the term **"near infrared-responsive photosensitizer"** refers to a compound which undergoes a chemical reaction by absorption of near infrared light, in particular at one or more specific wavelength corresponding to the absorption band or one of the absorption bands of the near infrared-responsive photosensitizer. A near infrared-responsive photosensitizer is a photo-responsive compound. Near infrared light refers to a light displaying a wavelength in the region of the spectrum extending from 600 nm to 2500 nm, in particular from 600 nm to 1200 nm, more particularly from 600 nm to 800 nm. In particular, a near infrared-responsive photosensitizer products chemical species, in particular oxidizing species, for example singlet oxygen (¹O₂), when exposed to near infrared light at the absorption wavelength of said photosensitizer.

As used herein, the term **"probe"** refers to a compound designed to generate a detectable MRI signal, in particular that incorporate MRI responsive atoms such as ¹³C, ²H (deuterium), ³¹P, or ¹⁹F. In particular, a probe may generate a ¹⁹F-MRI signal, a ¹³C-MRI signal, a ²H-MRI signal and/or a ³¹P-MRI signal. A probe may be selected according to the type of MRI used. For instance, a probe for ¹⁹F-MRI may be designed to generate a detectable ¹⁹F-MRI signal, in particular incorporates ¹⁹F atoms. Such probes may be commercially available.

As used herein, the term **"fluorinated"** refers to chemical compounds having at least one fluorine atom, for example molecules having at least one carbon - fluorine bond, in particular to chemical compounds having at least one perfluorinated moiety.

As used herein, the term **"perfluorinated"** and **"perfluorocarbon"** refers to chemical compounds that are analogs of hydrocarbons wherein all hydrogen atoms in the hydrocarbon are replaced with fluorine atoms. Perfluorinated molecules can also contain a number of other atoms, including bromine, chlorine, and oxygen. A bromine substituted perfluorocarbon is a perfluorocarbon wherein one or more of the fluorine atoms have been replaced with a bromine atom. A chlorine substituted perfluorocarbon is a perfluorocarbon wherein one or more of the fluorine atoms have been replaced with a chlorine atom. Examples of perfluorocarbons which may be suitable as a hydrophobic perfluorinated moiety include those selected from perfluoroalkanes, perfluoroalkylamines, perfluoro-crown-ethers, perfluorinated alcohols, perfluorohaloalkanes, perfluorinated carboxylic acids, perfluorinated azides, perfluorinated thiols, perfluorinated alkenes, perfluorinated alkynes, perfluorinated acrylates, and perfluorinated esters.

Examples of hydrophobic perfluorinated moieties/groups may be selected from the group consisting of: perfluorinated substituted C₁-C₃₀ alkyl groups, perfluorinated unsubstituted C₁-C₃₀ alkyl groups, perfluorinated substituted alkoxy groups, perfluorinated unsubstituted alkoxy groups.

As used herein, the term **"group"** may refer to a functional group of a chemical compound. For the purpose of the present disclosure, the terms **"group"** and **"moiety"** may be used interchangeably. Groups of the present compounds refer to an atom or a collection of atoms that are a part of the compound. Groups of the present invention may be attached to other atoms of the compound via one or more covalent bonds. Groups may also be characterized with respect to their valence state. The present term may thus also include groups characterized as monovalent, divalent, trivalent, etc. valence states.

As used herein, the term **"substituted"** refers to a compound wherein a hydrogen is replaced by another functional group.

As used herein, the term **"polymer"** refers to a molecule comprising a plurality of repeating chemical groups, typically referred to as monomers.

As used herein, the term **"copolymer",** also commonly referred to as a heteropolymer, is a polymer formed when two or more different types of monomers are linked in the same polymer.

As used herein, the term **"block copolymer"** refers to a type of copolymer comprising blocks or spatially segregated domains, wherein different domains comprise different polymerized monomers. In a block copolymer, adjacent blocks are constitutionally different, i.e. adjacent blocks comprise constitutional units derived from different species of monomer or from the same species of monomer but with a different composition or sequence distribution of constitutional units. Different blocks (or domains) of a block copolymer may reside on different ends of a polymer (e.g. [A] [B]), or may be provided in a selected sequence ([A][B][A][B]).

As used herein, the term **"diblock copolymer"** refers to block copolymers having two different chemical blocks. **"triblock copolymer"** refers to block copolymers having three different chemical blocks.

Block copolymers may include block copolymers having a first block comprising a first polymer such as a PEG polymer, for example a PEG polymer having 8 to 300 monomers, a second polymer, an intermediate block such as a fluorocarbon, including but not limited to, a fluorocarbon such as a fluorinated or perfluorinated alkane, and a third interior hydrophobic block. Block copolymers of the present invention are capable of undergoing self-assembly to make an external layer of a nanovector.

As used herein, the term **"block copolymer"** may thus include compounds comprising at least a first block, said first block optionally comprising or consisting of a first polymer such as PEG polymer, and at least a second block, said second block optionally comprising or consisting of a second polymer. The term "block copolymer" may also include functionalized block copolymers, such as copolymers having additional moieties.

As used herein, the term **"pharmaceutically acceptable"** refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

As used herein, the term "**(C₁-Cₓ)alkyl group"** respectively refers to a C₁-Cₓ normal, secondary or tertiary monovalent saturated hydrocarbon radical, for example (C₁-C₆)alkyl group. Examples are, but are not limited to, methyl, ethyl, propyl, n-propyl, isopropyl, butyl, isobutyl, sec-butyl, tert-butyl, pentyl, isopentyl, hexyl and isohexyl groups, and the like.

As used herein, the term **"(C₂-Cₓ)alkenyl group"** refers to a hydrocarbon radical which comprises at least one double bond, for example (C₂-C₆)alkenyl group. Examples are, but are not limited to, ethylenyl, propylenyl, butylenyl, pentylenyl and hexenyl groups, and the like.

As used herein, the term **"(C₁-Cₓ)alkoxy"** refers to a -O-(C₁-Cₓ)alkyl or -O-(C₃-Cₓ)cycloalkyl moiety, wherein alkyl and cycloalkyl are as defined above, for example (C₁-C₆)alkoxy. Examples are, but are not limited to, methoxy, ethoxy, 1-propoxy, 2-propoxy, cyclopropoxy, butoxy, tert-butoxy and pentoxy.

As used herein, the term **"(C₅-C₁₁)aryl group"** refers to a hydrocarbon aromatic ring. Examples are, but are not limited to phenyl, naphtyl and indenyl groups, and the like.

As used herein, the term **"(C₃-C₈)cycloalkyl group"** refers to a monocyclic saturated hydrocarbon, from 3 to 8 carbon atoms, saturated or partially unsaturated and unsubstituted or substituted. Examples of monocyclic rings are, but are not limited to, cyclopropyl, cyclobutyl, cyclopentyl, cyclohexyl, cycloheptyl and cyclooctyl.

As used herein, the term **"(C₅-C₁₁)heteroaryl group"** refers to a monocyclic aromatic or a bicyclic group wherein one to three carbon atom is replaced by a heteroatom, such as nitrogen, oxygen or sulphur. By way of examples of monocyclic aromatic ring of heteroaryl groups, more particulary as a (C₅-C₆)heteroaryl group, mention may be made of, but not limited to: oxazolyl, isoxazolyl, pyridyl, pyrimidinyl, pyridazinyl, triazinyl, pyrazinyl, oxadiazolyl, furanyl, pyrazolyl, thiazolyl, isothiazolyl, thiadiazolyl, imidazolyl, triazolyl and the like. In the framework of the present invention, the heteroaryl is advantageously pyridyl, imidazolyl, pyrazinyl, furanyl, thiazolyl, pyrazolyl, thiadiazolyl, pyridazinyl and pyrimidinyl, and in particular pyridyl, pyrazinyl, pyrimidinyl or pyridazinyl. By way of examples of bicyclic aromatic ring of heteroaryl groups, mention may be made of indolyl, isoindolyl, indolinyl, isoindolinyl, quinolinyl or isoquinolinyl.

As used herein, the term **"(C₃-C₈)heterocycloalkyl group",** as used herein, refers to a (C₃-C₈)cycloalkyl group wherein one or two of the carbon atoms are replaced with a heteroatom such as oxygen, nitrogen or sulphur, and more particularly with at least one nitrogen atom. Such heterocycloalkyl group may be saturated or partially saturated and unsubstituted or substituted. Examples are, but are not limited to piperazinyl, piperidinyl, pyrrolidinyl, aziridinyl, oxanyl, oxetanyl, tetrahydropyranyl, morpholinyl, tetrahydrofuranyl, oxazolidinyl, oxepanyl, diazepanyl, dioxanyl and tetrahydrothiopyranyl, and more particularly pyrrolidinyl, piperidinyl, morpholinyl, tetrahydropyranyl or one tetrahydrofuranyl group.

As used herein, an aromatic ring means, according to Hückel's rule, that a molecule has 4n + 2 π-electrons.

As used herein, the term **"halogen"** is understood to mean chlorine, fluorine, bromine, or iodine, and in particular denotes chlorine, fluorine or bromine.

As used herein, the terms **"chemically equivalent fluorine atoms"** relates to fluorine atoms presenting the same chemical environment and providing a single signal, in other terms the same signal for all the atoms, in NMR/MRI at a single frequency for imaging.

As used herein, the terms **"targeting agent"** relates to molecule designed to bind selectively to a specific biological target, such as a protein, enzyme, receptor, or nucleic acid, within a living organism. In particular, targeting agents are used to direct other entities, such as nanovectors, to specific cells, tissues, or molecular structures.

As used herein, the term **"subject"** refers to an animal, including, but not limited to a human and non-human mammal, including a primate (e.g., human), cow, sheep, goat, horse, dog, cat, rabbit, rat, or mouse. The terms "subject" and "patient" are used interchangeably herein in reference, in particular, to a mammalian subject, such as a human.

The terms **"tumor", "neoplasm", "proliferative disorder or disease"** and **"neoplastic disorder or disease"** are used interchangeably herein and are meant to refer to unwanted cell proliferation of one or more subset of cells in a multicellular organism resulting in harm (i.e., discomfort or decreased life expectancy) to the multicellular organisms. In certain embodiments, a tumor can be benign (non-invasive) or malignant (invasive).

As used herein, the term **"cancer"** is meant to refer to a malignant neoplasm, which is characterized by uncontrolled cell proliferation where cells have lost their normal regulatory controls that would otherwise govern the rate of cell growth. These unregulated, dividing cells can spread throughout the body and invade normal tissues in a process referred to as "metastasis". In the absence of other indications, the term is not construed to apply solely to one type of cancer, and may thus encompass those selected from: colorectal cancer, pancreatic cancer, lung cancer including non-small cell lung cancer, breast cancer, bladder cancer, gall bladder cancer, thyroid cancer, melanoma, liver cancer, uterine/cervical cancer, oesophageal cancer, kidney cancer, ovarian cancer, prostate cancer, head and neck cancer, and stomach cancer.

### Abbreviation

| | |
|---|---|
| Boc | tert-butyloxycarbonyl |
| CMC | critical micelle concentration |
| DCM | dichloromethane |
| DIAD | diisopropylazodicarboxylate |
| DLS | dynamic light scattering |
| DMF | dimethylformamide |
| EDCI | 1-ethyl-3-(3-dimethylaminopropyl)carbodiimide |
| equiv. | equivalent |
| HFIP | 1,1,1,3,3,3-Hexafluoro-2-propanol |
| min | minute(s) |
| mL | milliliter |
| mM | mmol/L : millimoles per litre |
| mmol | millimoles |
| µmol | micromoles |
| NHS | *N*-hydroxysuccinimide |
| NMR | nuclear Magnetic Resonance |
| PEG | polyethylene glycol |
| PFOA | perfluorooctanic acid |
| PFTD | perfluorotetradecanol |
| PPa | pyropheophorbide-a |
| TEM | transmission electron microscopy |
| THF | tetrahydrofuran |
| TLC | thin layer chromatography |

### EXAMPLES

### Materials & Methods

### General

Unless otherwise specified, chemicals were purchased from Sigma-Aldrich and used without further purification. Reactions were carried out under nitrogen using dry solvents, unless otherwise stated. Flash chromatography was carried out on Kieselgel 60 (230-240 mesh, Merck) and analytical TLC was performed on Merck precoated silica gel (60 F254). NMR spectra were recorded on a Bruker AVANCE DPX 400 spectrometer. ¹H NMR spectra were recorded at 400 MHz, ¹³C NMR at 100 MHz, and ¹⁹F at 376 MHz. Mass spectra were recorded using a Waters Micromass ZQ 2000 ESI spectrometer. The Critical Micelle Concentration was measured using a K10 Krüss tensiometer. Dynamic Light Scattering size measurements were performed on a VascoFlex instrument from Cordouan Technologies equipped with a 450 nm laser diode or on a Malvern Zetasizer Nano ZS (He-Ne laser 633 nm). Transmission Electron Microscopy (TEM) was carried out with a Philips CM12 microscope operated at 80 kV.

### Synthesis of Amphiphile PFTD-PEG of formula (I')

**Amphiphilic PFTD-PEG (I')** was obtained in two steps as described below and following scheme 1. First, monomethylether PEG (*MW* = 2000 Da) was activated into the corresponding tosylate **intermediate (1)** in 90% yield. Treatment of intermediate (1) with perfluorotetradecanol (PFTD) under basic conditions (NaH) led to the formation of **PFTD-PEG (I')** in yield (86%).

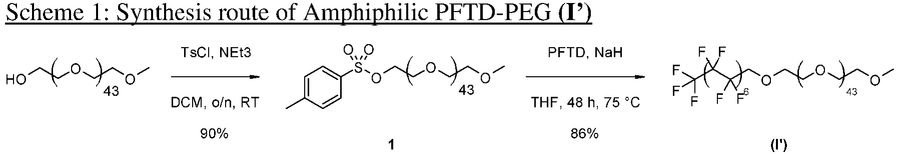

**Synthesis of intermediate (1).** Under a nitrogen atmosphere, polyethylene glycol monomethyl ether (Mn 2000, 3 g, 1.5 mmol, 1 equiv.) was dissolved in DCM (30 mL) and then sequentially treated with NEt₃ (1.04 mL, 7.5 mmol, 5 equiv.) followed by *p*-tosyl chloride (1.43 g, 7.5 mmol, 5 equiv.). The resulting solution was stirred for 48 h at room temperature and subsequently quenched with 5% HCl and brine. After collecting the organic phase, it was dried over MgSO₄, filtered, and concentrated under vacuum. Purification was achieved by precipitating the product with Et₂O at -20°C for 30 min. The tosylated PEG₂₀₀₀ monomethyl ether **intermediate (1)** was obtained as a white solid (2.86 g, 90%).

**¹H-NMR** (400 MHz, CDCl₃) *δ* (ppm) 7.82 (d, *J* = 8.2 Hz, 2 H), 7.33 (d, *J* = 8.2 Hz, 2 H), 4.15 (t, *J =* 4.6 Hz, 2 H), 3.44-3.70 (m, 10 H), 3.35 (s, 3 H), 2.41 (s, 3 H).

**¹³C-NMR** (100 MHz, D₂O) *δ* (ppm) 69.5 (multiple C), 58.0, 36.5.

**Synthesis of Amphiphilic PFTD-PEG (I').** Under a nitrogen atmosphere, sodium hydride (0.18 mg, 60% in oil, 4 equiv.) dissolved in THF (5 mL) was slowly introduced to perfluorotetradecanol (3 g, 4.40 mmol, 4 equiv.) in THF (50 mL). The resulting mixture was stirred at reflux for 30 min, following which the tosylated PEG₂₀₀₀ monomethyl ether **intermediate (1)** (2.5 g, 1.16 mmol, 1 equiv.) mentioned above was added. The reaction mixture was then stirred for 2 days at 75 °C. Afterward, THF was removed by evaporation, and the residual oil was dissolved in dichloromethane (DCM) and filtered through Celite^{®}. The **Amphiphilic PFTD-PEG (I')** was obtained as a white solid (2.08 g, 68%) through precipitation with diethyl ether (Et₂O) at -20 °C for 30 min. The molecular weight of the amphiphilic PFTD-PEG (I') is about 2680 Dalton.

**¹H-NMR** (400 MHz, D₂O) *δ* (ppm) 4.07 (t, 2H), 3.55 (s, 186H), 3.23 (s, 3H).

**¹³C-NMR** (100 MHz, D₂O) *δ* (ppm) 69.5 (multiple C).

**¹⁹F-NMR** (376 MHz, D₂O) *δ* (ppm) -123.16, -83.38.

**Mass** (MeOH) : ESI(+), *m*/*z* = 1327.5 [M+2H]²⁺

### Synthesis of ¹⁹F MRI probe (PERFECTA) of formula (IV)

**PERFECTA (IV)** was synthesized in one step as described below and following scheme 2, according to a procedure described in Tirotta et al., J. Am. Chem. Soc. 2014, 136, 8524, from the Mitsunobu reaction between pentaerythritol and fluorinated tert-butanol.

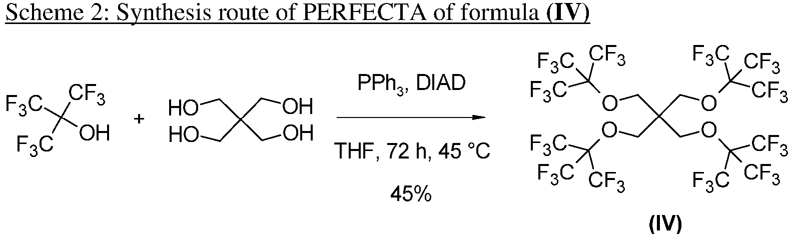

**Synthesis of PERFECTA (IV).** Under a nitrogen atmosphere at 0 °C, a solution of pentaerythritol (0.14 g, 1.03 mmol, 1 equiv.) and triphenylphosphine (1.6 g, 6.14 mmol, 6 equiv.) in anhydrous THF (10 mL) was stirred, to which diisopropylazodicarboxylate (DIAD, 1.22 mL, 6.14 mmol, 6 equiv.) was added dropwise. After 30 min at room temperature, perfluoro-tert-butanol (0.86 mL, 6.14 mmol, 6 equiv.) was added in one portion. The mixture was then stirred at 45 °C for 72 h until a fluorinated layer formed. After cooling to 0 °C, the mixture was filtered and washed with THF. The resulting product was obtained as a white solid (0.46 g, 45%).

**¹H-NMR** (400 MHz, CDCl₃/HFIP) *δ* (ppm) 4.45-4.36 (m, HFIP), 4.17 (s), 3.59 (s, HFIP). **¹³C-NMR** (100 MHz, CDCl₃) *δ* (ppm) 120.6 (q, *J =* 280 Hz), 119.1 (HFIP), 79.5 (m), 69.5 (HFIP), 65.5, 46.0.

**¹⁹F-NMR** (376 MHz, CDCl₃) *δ* (ppm) -76.14 (s, HFIP), -70.85 (s).

### Synthesis of fluorinated ferrocene derivative (FFc) of formula (II')

**FFc (II')** was synthesized as described below and following scheme 3. FFc of formula **(II')** was obtained by peptide coupling between **ferrocene dicarboxylate intermediate 6** (in its activated diester form) and **fluorinated amine intermediate 5.** Upon reaction in chloroform at room temperature in the presence of triethylamine, FFc **(II')** was obtained in 35% yield.

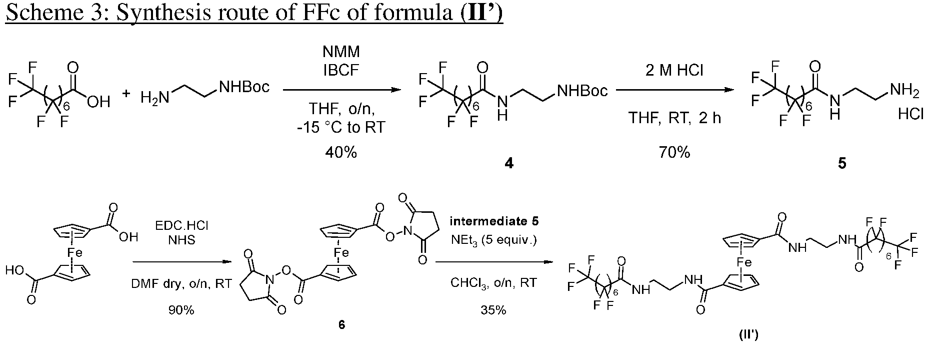

**Synthesis of intermediate 4.** Under a nitrogen atmosphere, perfluorooctanic acid (PFOA, 1 g, 2.41 mmol, 1 equiv.) and *N*-methyl morpholine (0.29 mL, 2.64 mmol, 1.1 equiv.) were dissolved in dry THF (20 mL) and cooled to -15 °C. Isobutyl chloroformate (0.34 mL, 2.62 mmol, 1.1 equiv.) was added dropwise to the stirred solution, followed by the addition of *N*-Boc-ethylenediamine (0.38 g, 2.41 mmol, 1 equiv.) after 10 min. The reaction mixture was stirred for 2 h and then concentrated under vacuum and diluted with ethyl acetate. After washing the organic phase with 1 M HCl (aq), water, and brine, it was dried with MgSO₄, filtered, and concentrated. The crude product was purified by flash column chromatography using a gradient of cyclohexane/ethyl acetate from 8:2 to 7:3 to yield **intermediate 4** as a white solid (536 mg, 40%).

**¹H NMR** (400 MHz, CDCl₃) *δ*(ppm) 3.47 (dd, *J =* 10.0, 4.7 Hz, 2H), 3.38 (dd, *J =* 10.1, 4.6 Hz, 2H), 1.44 (s, 9H).

**Mass** (MeOH): ESI(+) *mlz =* 457 [M-Boc+H]⁺, ESI(-) *mlz* = 555 [M-H]⁻

**Synthesis of intermediate 5.** A solution of HCl in dioxane (4 M, 1 mL) was added dropwise to a stirred solution of **intermediate 4** (536 mg, 0.96 mmol, 1.0 equiv.) in THF (10 mL). After stirring for 17 h at room temperature, the reaction mixture was dried under vacuum. The resulting solid was triturated with diethyl ether (Et₂O) and then filtered to obtain **intermediate 5** as a white solid (331 mg, 70% yield).

**¹H NMR** (400 MHz, DMSO-d6) *δ* (ppm) 3.46 (dd, *J* = 11.6, 6.1 Hz, 2H), 2.93 (t, *J =* 6.6 Hz, 2H).

**Mass** (MeOH) : ESI(+) *mlz* = 457 [M+H]⁺, ESI(-) *mlz* = 455 [M-H]⁻

**Synthesis of intermediate 6.** To a solution of 1,1-ferrocene dicarboxylic acid (400 mg, 1.46 mmol, 1 equiv.) in DMF (15 mL) were added 1-ethyl-3-(3-dimethylaminopropyl)carbodiimide hydrogen chloride (EDCI·HCl, 680 mg, 3.50 mmol, 2.4 equiv.), and N-hydroxysuccinimide (NHS, 380 mg, 3.36 mmol, 2.3 equiv.). The mixture was stirred at room temperature overnight. The reaction mixture was concentrated, and then redissolved in DCM and washed successively with a NaHCOs saturated solution, water and brine. The combined organic phases were dried over MgSO₄, and concentrated *in vacuo* to give the desired **intermediate 6** as a red solid (578 mg, 85%).

**¹H NMR** (400 MHz, DMSO-d₆) *δ* (ppm) 5.16 (m, 4H), 4.92 (m, 4H), 2.88 (m, 8H).

**¹³C NMR** (100 MHz, DMSO-d₆) *δ*(ppm) 170.5, 165.8, 75.7, 72.5, 66.2, 25.5.

**Mass** (MeOH) : ESI(+), *m*/*z* = 491.6 [M+Na]⁺

**Synthesis of FFc (II'). Intermediate 6** (105 mg, 0.22 mmol, 1 equiv.) was dissolved in chloroform (10 mL) and then treated with NEt₃ (0.16 mL, 1.15 mmol, 5 equiv.), followed by the addition of the **fluorinated amine intermediate 5** (0.33 g, 0.67 mmol, 3 equiv.). The resulting solution was stirred for 2 days at room temperature and then quenched with 5% HCl and brine. After collecting the organic phase, it was dried over MgSO₄, filtered, and concentrated *in vacuo.* The crude product was purified by flash column chromatography using a gradient of cyclohexane/ethyl acetate from 3:7 to 2:8, yielding **FFc** (**II**') as a yellow solid (101 mg, 40%).

**¹H NMR** (400 MHz, DMSO-d6) *δ*(ppm) 4.72-4.67 (m, 4H), 4.37-4.32 (m, 4H), 3.40 (dd, *J=* 11.4, 5.7 Hz, 4H), 3.38 (dd, *J* = 10.6, 5.1 Hz, 4H).

**Mass** (MeOH) : ESI(+), *mlz* = 1151.1 [M+H]⁺, *mlz =* 1173 [M+Na]⁺

### Synthesis of near infrared-responsive photosensitizer (NIR-PS) of formula (III')

**NIR-PS (III')** was synthesized by peptide coupling between **fluorinated amine intermediate 5** and the **activated ester of pyropheophorbide-a intermediate 8.** Upon reaction in DMF at room temperature in the presence of triethylamine, **NIR-PS (III')** was obtained in 66% yield.

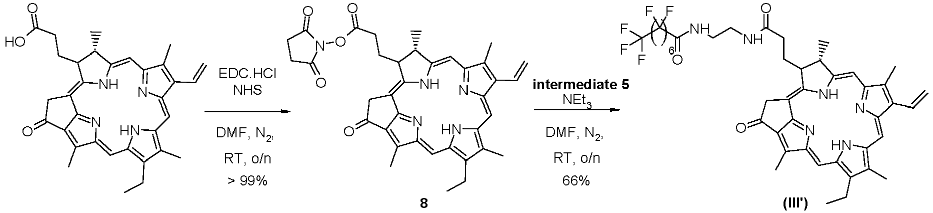

**Synthesis of intermediate 8:** To a solution of pyropheophorbide-a (PPa, 20 mg, 0.04 mmol, 1 equiv.) in DMF (5 mL), were added 1-ethyl-3-(3-dimethylaminopropyl)carbodiimide hydrogen chloride (EDCI·HCl, 71 mg, 0.37 mmol, 10 equiv.), and N-hydroxysuccinimide (NHS, 45 mg, 0.37 mmol, 10 equiv.). The mixture was stirred in the dark at room temperature overnight. The reaction mixture was poured in water and extracted with DCM. It was washed successively with a NaHCOs saturated solution and brine. The combined organic phases were dried over MgSO₄, and concentrated *in vacuo* to give the desired **intermediate 8** as a dark solid (20 mg, 80%).

**¹H NMR** (400 MHz, CDCl₃) *δ*(ppm) 9.53 (s, 1H), 9.42 (s, 1H), 8.58 (s, 1H), 8.02 (dd, *J* = 17.7, 11.6 Hz, 1H), 6.30 (dd, *J* = 17.8, 1.5 Hz, 1H), 6.18 (dd, *J* = 11.6, 1.5 Hz, 1H), 5.20 (q, *J* = 19.8 Hz, 2H), 4.57-4.48 (m, 1H), 4.47-4.40 (m, 1H), 3.75-3.66 (m, 5H), 3.42 (s, 3H), 3.26 (s, 3H), 2.87 (s, *J =* 6.5 Hz, 5H), 2.69-2.59 (m, 1H), 2.41-2.26 (m, 1H), 1.82 (d, *J* = 7.3 Hz, 3H), 1.71 (t, *J* = 7.6 Hz, 3H).

**Mass** (MeOH): ESI(+), *m*/*z* = 633.0 [M+H]⁺

**Synthesis of NIR-PS (III'). Intermediate 8** (20 mg, 0.03 mmol, 1 equiv.) was dissolved in DMF (3 mL) and then treated with NEt₃ (78 µL, 0.09 mmol, 3 equiv.), followed by the addition of **fluorinated amine intermediate 5** (22 mg, 0.05 mmol, 1.6 equiv.). The resulting solution was stirred overnight at room temperature and then quenched with 5% HCl and brine. After collecting the organic phase, it was dried over MgSO₄, filtered, and concentrated *in vacuo.* The crude product was purified by flash column chromatography using a gradient of dichloromethane/methanol from 10:0 to 9:1, yielding **NIR-PS (III')** as a dark solid (20 mg, 69%).

**¹H NMR** (400 MHz, DMSO-d6) *δ* (ppm) 9.37 (s, 1H), 9.08 (s, 1H), 8.53 (s, *J* = 6.7 Hz, 1H), 7.99 (dd, *J* = 17.8, 11.5 Hz, 2H), 6.29 (dd, *J* = 17.8, 1.3 Hz, 1H), 6.18 (dd, *J* = 11.6, 1.3 Hz, 1H), 5.70 (t, *J* = 5.0 Hz, 1H), 5.20 (d, *J=* 19.7 Hz, 1H), 5.02 (d, *J* = 19.7 Hz, 1H), 4.53-4.44 (m, 1H), 4.35-4.27 (m, 1H), 3.76-3.67 (m, *J* = 6.1, 3.0 Hz, 1H), 3.66-3.53 (m, 1H), 3.52-3.45 (m, *J* = 14.3, 7.6 Hz, 1H), 3.40 (s, 3H), 3.23 (s, 3H), 3.20-3.08 (m, 5H), 3.06-2.96 (m, *J=* 14.5, 5.2 Hz, 1H), 2.89 (t, *J =* 6.4 Hz, 1H), 2.72-2.59 (m, 1H), 2.57-2.45 (m, 1H), 2.38-2.31 (m, *J* = 7.4 Hz, 1H), 2.27-2.16 (m, 1H), 2.01-1.90 (m, *J* = 14.7, 9.5, 5.6 Hz, 1H), 1.78 (d, *J =* 7.3 Hz, 3H), 1.58 (t, *J =* 7.5 Hz, 3H).

**¹⁹F-NMR** (376 MHz, CDCl₃) *δ* (ppm) -126.12, -122.63, -121.80, -119.72, -80.73, -75.89.

**Mass** (MeOH) : ESI(+) *mlz =* 974.2 [M+H]⁺, ESI(-) *mlz* = 972.1 [M-H]⁻

### Determination of the Critical Micellar Concentration (CMC) by surface tension measurement

Different dispersions of **PFTD-PEG (I')** were prepared at variable concentrations: 0.001, 0.005, 0.01, 0.05, 0.1, 0.2, and 0.1 mg mL⁻¹. Surface tension was measured (using K10 Krüss tensiometer) in triplicate for each concentration, which gave two straight lines. The intersection of both lines corresponds to the point at which **PFTD-PEG (I')** begin to self-assemble into micelles. From this concentration and higher, any added amphiphiles will form micelles.

### Micelles size analysis by Dynamic Light Scattering (DLS)

Dynamic light scattering (DLS) experiments were performed using a VascoFlex instrument from Cordouan Technologies using a laser diode (λ = 450 nm) at 20 °C or using a Malvern Zetasizer Nano ZS at 25 °C (He-Ne laser 633 nm).

Hydrodynamic diameters *Dₕ* were recorded using Dynamic light scattering (DLS) experiments described above for different micelle and/or nanovector formulations.

### Example 1. PFTD-PEG (I') micelles formulation and characterization

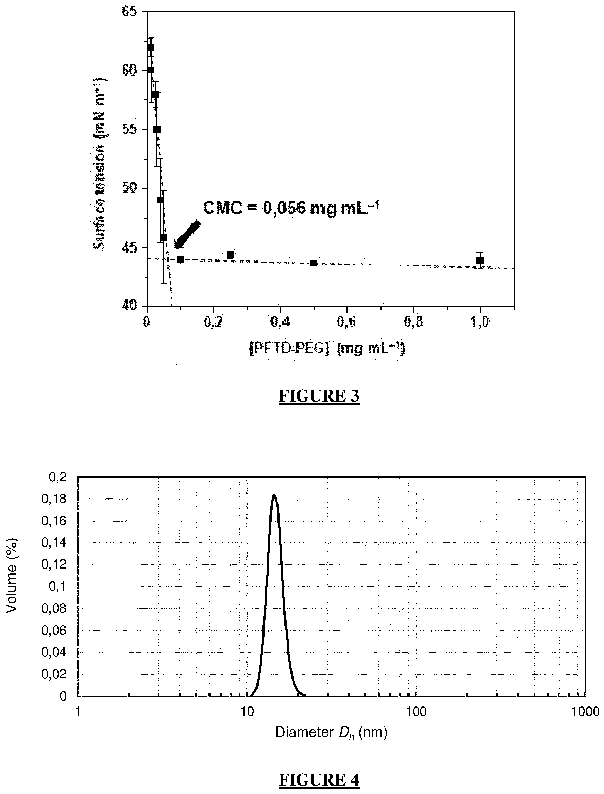

**PFTD-PEG (I') micelles** were assembled by simply dispersing **amphiphilic PFTD-PEG (I')** in water at a concentration higher than the corresponding Critical Micelle concentration (CMC) which was determined by surface tension measurements as described above and found to be 0.056 mg mL⁻¹ as represented in figure 3.

A 20 mg mL⁻¹ suspension of **amphiphilic PFTD-PEG (I')** in water was probe-sonicated for 10 min to yield a formulation of **PFTD-PEG (I') micelles** which were then analyzed by dynamic light scattering (DLS) as described above. The dynamic light scattering profile of **PFTD-PEG (I') micelles** is represented in figure 4. In figure 4, the average hydrodynamic diameter was found to be 14 nm. In addition, transmission electron microscopy (TEM) revealed monodisperse spherical objects with a size consistent with that measured by DLS.

### Example 2. Preparation of different formulations of nanovectors and/or micelles (# 1 to # 4)

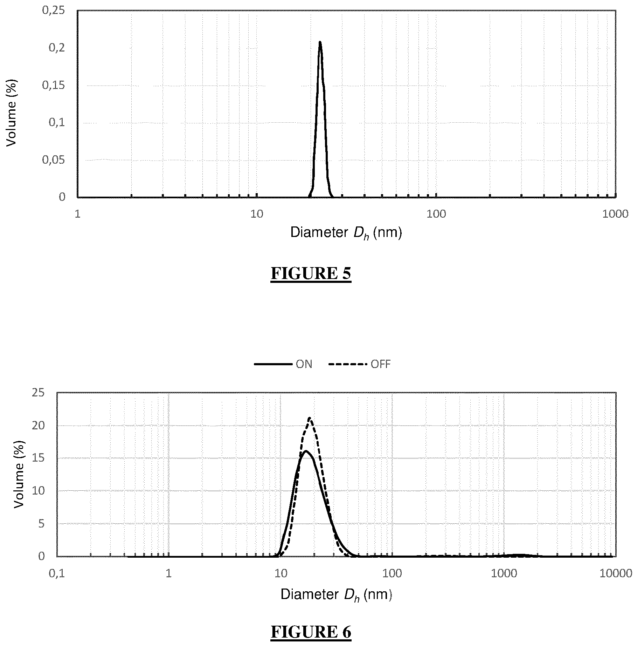

Different micelle formulations (# 1 to # 4) in table 1 below were prepared as follows. In table 1, the weight concentration (in mg mL⁻¹) of each compound in the formulation and their percentage by weight (wt%) relative to the total weight of the nanovector are provided, as well as the hydrodynamic diameters (*Dₕ*) determined by DLS as described above. Figures 5 and 6 respectively represent the DLS profiles of micelles in formulations # 2 and # 3 respectively. In figure 6, two DLS profiles are provided for nanovectors and/or micelles formulation which did not undergo any treatment such as blue light illumination, referred to as "ON state", and for nanovectors and/or micelles formulation exposed to illumination at a wavelength of 450 nm before measures (Irradiance = 140 mW cm⁻² at 5 cm from the lamp), referred to as "OFF state".

### Preparation of PFTD-PEG micelles outside the invention - formulation # 1

A solution containing 20 mg of **amphiphilic PFTD-PEG** was prepared in 1 mL of ultrapure water. This mixture was then subjected to ultrasound using an ultrasonic probe (300 ms pulse per second, 45% output) for 10 min, resulting in a final micelle concentration of 20 mg mL⁻¹. The solution was subsequently filtered through a 0.22 µm nylon membrane.

### PERFECTA@PFTD-PEG nanovectors outside the invention - formulation # 2

**PERFECTA (IV)** (20 mg) was introduced into a solution of **PFTD-PEG (I') micelles** in ultrapure water (1 mL - 20 mg mL⁻¹) as obtained by the preparation of PFTD-PEG micelles detailed previously and the mixture underwent sonication with an ultrasonic probe (300 ms pulse per second, output 45%) for 20 min. Subsequently, the suspension was filtered through a 0.22 µm membrane to eliminate insoluble aggregates. The encapsulation efficiency was measured to be 70%.

### PERFECTA/FFc@PFTD-PEG nanovectors according to the invention - formulation #3

**FFc** (**II**') (5 mg) and **PERFECTA (IV)** (20 mg) were added to a solution of **PFTD-PEG (I') micelles** in ultrapure water (1 mL - 20 mg mL⁻¹) as obtained by the preparation of PFTD-PEG micelles detailed previously and the mixture was sonicated with an ultrasonic probe (300 ms pulse per second, output 45%) for 20 min. The suspension was then filtered on a 0.22 µm membrane. PERFECTA **(IV)** encapsulation efficiency remains at 70% whilst that of FFc **(II')** was estimated at 100%. The nanovector obtained is illustrated on figure 1.

### PERFECTA/FFc/NIR-PS@PFTD-PEG nanovectors according to the invention - formulation # 4

**FFc (II')** (5 mg), **PERFECTA (IV)** (20 mg) and **NIR-PS** (**III**') (0.2 mg) were added to a solution of **PFTD-PEG (I') micelles** in ultrapure water (1 mL - 20 mg mL⁻¹) as obtained by the preparation of PFTD-PEG micelles detailed previously and the mixture was sonicated with an ultrasonic probe (300 ms pulse per second, output 45%) for 20 min. The suspension was then filtered on a 0.22 µm membrane. PERFECTA **(IV)** encapsulation efficiency remains at 70% whilst that of FFc (**II**') and NIR-PS (**III**') were estimated at 100%. The nanovector obtained is illustrated on figure 2.

**Table 1. Different micelle and/or nanovector formulations # 1 to # 4**

| **Formulation** | **# 1** | **# 2** | **# 3** | **# 4** |
|---|---|---|---|---|
| PFTD-PEG **(I')** | 20 mg mL⁻¹ | 20 mg mL⁻¹ | 20 mg mL⁻¹ | 20 mg mL⁻¹ |
| PERFECTA **(IV)** | - | 14 mg mL⁻¹ | 14 mg mL⁻¹ | 14 mg mL⁻¹ |
| | | 40 wt% | 40 wt% | 40 wt% |
| FFc **(II')** | - | - | 5 mg mL⁻¹ | 5 mg mL⁻¹ |
| | | | 20 wt% | 20 wt% |
| NIR-PS (**III**') | - | - | - | 0,2 mg mL⁻¹ |
| | | | | 1 wt% |
| Diameter (*Dₕ*) | 14 nm | 20 nm | 19 nm | 20 nm |

### Example 3. Cytotoxicity assessment

In order to evaluate the cytotoxicity of different micelles and/or nanovectors formulations and ensure that they are safe for medical imaging purposes, their potential cytotoxicity was evaluated using cytotoxicity assays. To this end, cancer cells, MC-38 and MCF7 cell lines, were incubated with increasing concentrations of micelles formulations and cell survival was measured after 72 h of contact as described in the Cytotoxicity assays above.

### Nanovectors and/or micelles formulations preparation

PFTD-PEG micelles formulation and PERFECTA/FFc@PFTD-PEG nanovectors formulation were prepared as described in example 2, respectively as formulation # 1 and formulation # 3.

FFc@PFTD-PEG nanovectors formulation was prepared as described in example 2 for formulation # 3 without the addition of PERFECTA **(IV).**

### Cytotoxicity assays

Human breast cancer cell line (MCF-7) and Murine colon carcinoma cell line (MC38) were used for *in vitro* experiments.

MCF-7 cells were cultured in medium with dulbecco's modified eagle's medium (DMEM), fetal bovine serum (FBS) 10% (v/v), Pen-Strep 1% (v/v) and sodium pyruvate (1 mM).

MC38 cells were routinely maintained in DMEM supplemented with 10% (v/v) Fetal Bovine Serum (PAA), glutamin (2 mM), non-essential amino acids (0.1mM), sodium pyruvate (1 mM), Hepes (10 mM), gentamycin sulfate (50 µg mL⁻¹), Penicllin (100 U mL⁻¹) and Streptomycin (100 µg mL⁻¹).

Cell proliferation/survival assay of cells exposed to micelles and/or nanovectors was conducted in the same way as described in the literature M.-D. Hoang et al., Nanoscale Adv. 2019, 1, 4331-4338.

In short, MCF-7 or MC38 cells suspended in culture medium were seeded into the wells of an optical 96-well culture plate (Costar #3904) at a final density of 1000 (MC38) and 1500 cells/well (MCF-7). Plates were then incubated for 6 h at 37 °C, 5% CO₂ to allow cell adhesion. Micelles and/or nanovectors formulations (or PBS as vehicle) were then diluted in PBS and immediately layered on top of the culture wells to achieve the indicated final concentrations, and plates were incubated for 72 h at 37 °C, 5% CO₂. In some assays, the plates were then illuminated with blue light at 450 nm using a Kessil blue LED lamp (Kessil A160WE Tuna Blue, intensity = 40 W) for 10 min. Cells were then fixed by addition of paraformaldehyde (4% [w/v] final) and nuclear DNA was fluorescently labeled by Hoechst 33342 (2 µg mL-1 final). After an overnight incubation at 4 °C, supernatants were removed by aspiration, replaced by 100 µL PBS, and plates were imaged on a high-content imaging device (Operetta, Perkin Elmer) in the blue channel (Ex. 360-400 nm, Em. 410-480 nm, 10 × magnification, 9 fields acquired/well). Using a high-content imaging analysis software (Harmony 3.0, Perkin-Elmer), DNA-labeled nuclei were segmented, and the absolute amount of nuclei per condition was quantitated. Results are expressed as the relative amount of cells in micelle-treated wells relative to the average amount of cells in the controls treated with vehicle (PBS).

### Results

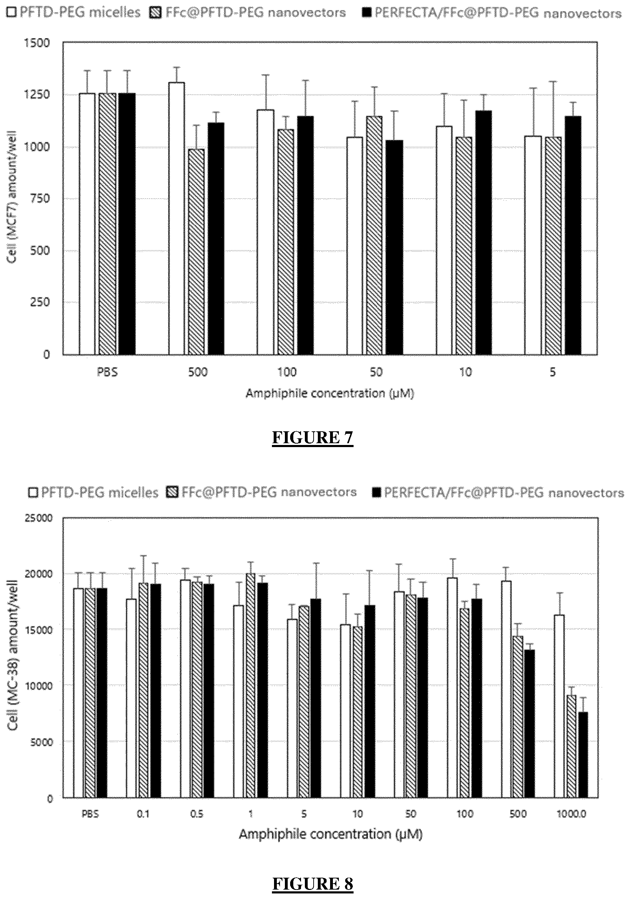

As shown in figures 7 to 9, nanovectors and/or micelles solutions corresponding to concentrations of PFTD-PEG (I') up to 100 µM showed no toxicity at all for both cell lines.

### Example 4. ¹⁹F-MRI and ¹H-MRI signal modulation studies

The nanovectors, which are micelles, were prepared as detailed in example 2 according to the formulations reported in table 2, where the molar concentrations in water (mM) are reported.

The samples for MRI acquisition were prepared by placing 750 µL of the nanovector formulations according to the invention (I1 to I5) or outside the invention (C1) in 750 µL transparent glass cylindrical vials, then placed in a 50 mL Falcon^{®} tube. All MRI experiments were performed with a 7 T Biospec preclinical scanner (Bruker) and a double-tuned (¹H/¹⁹F) volume coil from RAPID Biomedical.

### ¹⁹F MRI experiments

To determine the ¹⁹F T₁ and T₂ relaxation times of PERFECTA-loaded nanovectors, several parametric sequences were acquired on a tube filled with fluorinated micelles at 0.5 M. For T₁ estimation, an Inversion Recovery Fast Gradient Echo sequence (IR-FGE) with the following parameters was used: TR₁ = 5 ms; TR₂ = 10 s; TE = 2.5 ms; 9 segments; flip angle = 5°; 160 inversion times spaced by 40 ms from 40 ms to 6400 ms; voxel size = 0.4 mm × 0.4 mm × 10 mm; matrix = 72 × 72 × 2; acquisition time = 25 h 36 mn; 128 averages and 4 repetitions. After images reconstruction, the T₁ relaxation time was computed by fitting the signal intensity versus inversion time using the approach proposed by Deichmann *et al.*

(Magn. Reason. Med., 1999, 42:206-9). For T₂ estimation, a Multi Slice Multi Echo sequence (MSME) with the following parameters was used: TR = 2500 ms; TE = 5 ms; 128 echoes spaced by 5 ms from 5 ms to 640 ms; voxel size = 0.4 mm × 0.4 mm × 8 mm; matrix = 72 × 72 × 2; acquisition time = 6 h 24 min; 64 averages and 2 repetitions. After images reconstruction, the T₂ relaxation time was computed by fitting the signal intensity versus echo time using Bloch equations.

### ¹H MRI experiments

To determine the ¹H T₁ and T₂ relaxation times of PERFECTA-loaded nanovectors, several parametric sequences were acquired on a tube filled with fluorinated micelles at 0.5 M. For T₁ estimation, an IR-FGE sequence with the following parameters was used: TR₁ = 5 ms; TR₂ = 10 s; TE = 2.5 ms; 9 segments; flip angle = 5°; 160 inversion times spaced by 40 ms from 40 ms to 6400 ms; voxel size = 0.4 mm × 0.4 mm × 8 mm; matrix = 72 × 72 × 2; acquisition time = 12 h 48 mn; 64 averages and 4 repetitions. After images reconstruction, the T₁ relaxation time was computed by fitting the signal intensity versus inversion time using the approach proposed by Deichmann et al. (Magn. Reason. Med., 1999, 42:206-9). For T₂ estimation, a MSME sequence with the following parameters was used: TR = 2500 ms; TE = 5 ms; 122 echoes spaced by 5 ms from 5 ms to 610 ms; voxel size = 0.4 mm × 0.4 mm × 8 mm; matrix = 72 × 72 × 2; acquisition time = 6 h 24 min; 64 averages and 2 repetitions. After images reconstruction, the T₂ relaxation time was computed by fitting the signal intensity versus echo time using Bloch equations.

### Results

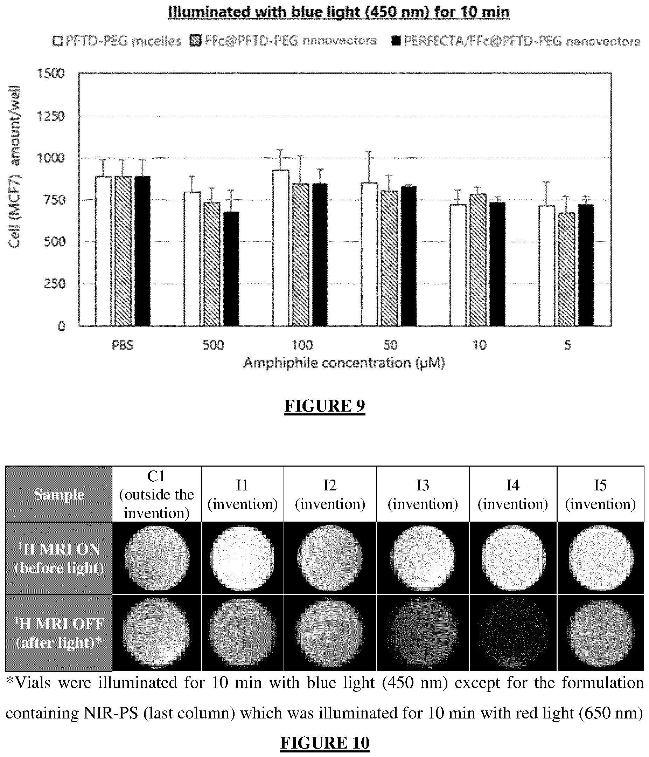

Table 3 and figure 10 present the results of ¹H MRI signals modulation studies, carried out *in vitro.*

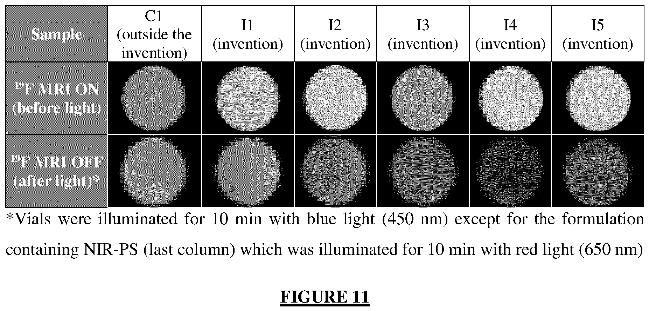

Table 4 and figure 11 present the results of ¹F MRI signals modulation studies, carried out *in vitro.*

The results obtained by MRI are labelled "ON" when the sample does not undergo any treatment such as blue light or red light illumination before MRI. The results obtained by MRI are labelled "OFF" after illumination as detailed here-after:
- C1 outside the invention which does not comprise organometallic complex, was illuminated for 10 min with blue light (450 nm);
- I1 to I4 according to the invention, were illuminated for 10 min with blue light (450 nm, 40 W, 140 mW cm⁻²) 10 min before MRI; and
- I5 according to the invention, was illuminated for 10 min with red light (650 nm, 40 W) 10 min before MRI.

**Table 2. Nanovector formulations**

| **Sample** | C1 (outside the invention) | I1 (invention) | I2 (invention) | I3 (invention) | I4 (invention) | I5 (invention) |
|---|---|---|---|---|---|---|
| **PFTD-PEG (I') (mM)** | 7.6 | 7.6 | 7.6 | 7.6 | 7.6 | 7.6 |
| **PERFECTA (IV) (mM)** | 13.2 | 13.2 | 13.2 | 13.2 | 13.2 | 13.2 |
| **FFc (II') (mM)** | / | 0.92 | 1.93 | 3.08 | 4.35 | 4.35 |
| **NIR-PS (III') (mM)** | / | / | / | / | / | 0.10 |

**Table 3. Results of ¹H-MRI**

| **Sample** | C1 (outside the invention) | I1 (invention) | I2 (invention) | I3 (invention) | I4 (invention) | I5 (invention) |
|---|---|---|---|---|---|---|
| **T₁ ON (ms)** | 2275 | 2109 | 2278 | 2278 | 2328 | / |
| **T₂ ON (ms)** | 1105 | 869 | 552 | 548 | 802 | / |
| **T₁ OFF (ms)** | 2196 | 1947 | 2026 | 1722 | 711 | / |
| **T₂ OFF (ms)** | 1076 | 440 | 50 | 27 | 7 | 71 |

**Table 4. Results of ¹F-MRI**

| **Sample** | C1 (outside the invention) | I1 (invention) | I2 (invention) | I3 (invention) | I4 (invention) | I5 (invention) |
|---|---|---|---|---|---|---|
| **T₂ ON (ms)** | 150 | 129 | 99 | 96 | 118 | / |
| **T₂ OFF (ms)** | 154 | 72 | 25 | 28 | 10 | 69 |

### Conclusion

As demonstrates in tables 3 and 4, the relaxation time T₂ OFF for ¹H-MRI and ¹⁹F-MRI, is significantly reduced for the samples I1 to I4 according to the invention compared to the sample C1 outside the invention. Such reduction is linked to the loss of MRI signal visible on figures 10 and 11.

Studies carried out *in vitro,* following the above-mentioned experiments, demonstrate that, upon blue light illumination, ¹H and ¹⁹F MRI signals of the nanovectors comprising PFTD-PEG **(I'),** PERFECTA **(IV)** and FFc **(II')** can be attenuated compared to a nanovector not comprising FFc (**II**'). Furthermore, the attenuation effect is correlated to the amount of FFc **(II')** encapsulated in the nanovectors.

Furthermore, the inclusion of NIR-PS (**III**') in addition to FFc **(II')** for the sample I5 allows the attenuation of the signal under near infrared light illumination, which is, for example, suitable for *in vivo* experiments, as shown in table 3 and figure 10 for ¹H-MRI and in table 4 and figure 11 for ¹⁹F-MRI.

Thus, the nanovectors according to the invention is responsible for the modulation of ¹H and ¹⁹F MRI signals which can be controlled, in particular decreased, on demand thanks to light illumination applied on a selective zone.

## Claims

1. Nanovector comprising:
- an external layer comprising a plurality of amphiphilic molecules, said amphiphilic molecules comprising at least one hydrophobic moiety linked to at least one hydrophilic moiety, and
- a core comprising at least one organometallic complex having a paramagnetic property that can be activated by oxidation, said organometallic complex being functionalized by at least one hydrophobic moiety,
the oxidation of the organometallic complex being controllable by light, the light having in particular a wavelength comprised between 300 nm and 1200 nm, more particularly between 400 and 800 nm, even more particularly between 400 nm and 700 nm.

2. Nanovector according to claim 1, wherein:
- said organometallic complex is photo-responsive; and/or
- said core further comprises at least one near infrared-responsive photosensitizer suitable to produce oxidizing species when exposed to near infrared light and being functionalized by at least one hydrophobic moiety.

3. Nanovector according to claim 1 or 2, wherein said hydrophilic moiety comprises a polyethylene glycol (PEG) containing between 8 and 100 ethoxy monomers, in particular containing between 30 and 60 ethoxy monomers, and more particularly containing between 40 and 50 ethoxy monomers.

4. Nanovector according to any of the preceding claims, wherein the amphiphilic molecules comprise at least one hydrophobic perfluorinated moiety linked to said hydrophilic moiety, in particular the plurality of amphiphilic molecules comprises, notably consists of, compounds of formula (I): 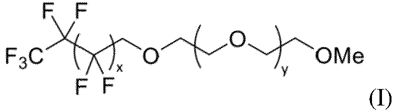
wherein x ranges from 8 to 15,
wherein y ranges from 8 to 100,
or mixtures of compounds of formula (I),
more particularly comprises, notably consists of, compounds of formula (I'):

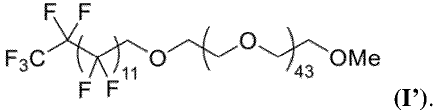

5. Nanovector according to any of the preceding claims, wherein the organometallic complex comprises at least one metallocene group, in particular comprises at least one ferrocenyl group, said organometallic complex being optionally functionalized by at least one hydrophobic perfluorinated moiety, more particularly the organometallic complex is a compound of formula (II): 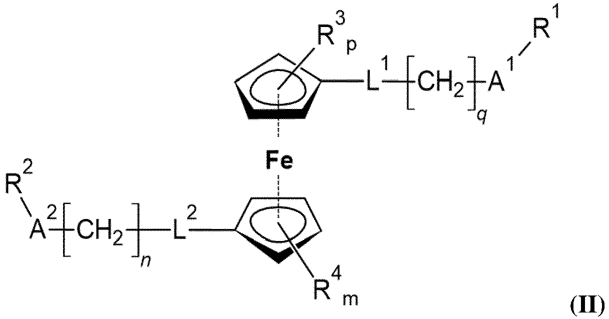 wherein:
- L¹ and L² independently represent a single bond, a -O- group, a -NH- group, a -C(=O)-group, a -C(=O)-O- group, a -O-C(=O)- group, a -NH-C(=O)- group, or a -C(=O)-NH-group, in particular L¹ and L² independently represent a -C(=O)-O- group being linked to said ferrocenyl group via its carbon atom and to the group [CH₂]_{q} or [CH₂]ₙ via the oxygen atom, or a -C(=O)-NH- group being linked to said ferrocenyl group via its carbon atom and to the group [CH₂]_{q} or [CH₂]ₙ via its nitrogen atom;
- n and q are independently equal to 0, 1, 2, 3 or 4;
- A¹ and A² independently represent a single bond, a -O- group, a -NH- group, a -C(=O)-group, a -C(=O)-O- group, a -O-C(=O)- group, a -C(=O)-NH- group, or a -NH-C(=O)-group, in particular A¹ and A² independently represent a -C(=O)-O- group, a -O-C(=O)-group, a -NH-C(=O)- group, or a -C(=O)-NH- group;
- R¹ and R² independently represent a hydrophobic moiety selected from a substituted or unsubstituted, saturated or unsaturated, aliphatic C₄-C₃₆ chain-containing moiety and a hydrophobic perfluorinated moiety, in particular a hydrophobic perfluorinated moiety, more particularly a -(CF₂)ₜ-CF₃ group wherein t is equal to 1 to 25;
- p and m are identical or different, and equal to 0, 1, 2 or 3; and
- when p and/or m is(are) strictly greater than 0, R³ and R⁴ independently represent a (C₁-C₆)alkyl group, a (C₂-C₆)alkenyl group, a (C₅-C₁₁)aryl group, a (C₃-C₈)cycloalkyl group, a (C₅-C₁₁)heteroaryl, (C₃-C₈)heterocycloalkyl group or a halogen atom, in particular selected from an iodine atom, a chlorine atom and a bromine atom;
or a mixture of compounds of formula **(II),**
in particular wherein:
- L¹ and L² independently represent a -C(=O)-O- group being linked to said ferrocenyl group via its carbon atom and to the group [CH₂]_{q} or [CH₂]ₙ via the oxygen atom, or a - C(=O)-NH- group being linked to said ferrocenyl group via its carbon atom and to the group [CH₂]_{q} or [CH₂]ₙ via its nitrogen atom;
- n and q are independently equal to 1, 2 or 3;
- A¹ and A² independently represent a -C(=O)- group, a -C(=O)-O- group, a -O-C(=O)-group, a -C(=O)-NH- group, or a -NH-C(=O)- group, in particular a -C(=O)- group, a - C(=O)-NH- group, or a -NH-C(=O)- group;
- R¹ and R² independently represent a hydrophobic perfluorinated moiety, in particular a -(CF₂)ₜ-CF₃ group wherein t is equal to 1 to 25, more particularly equal to 3 to 15, even more particularly equal to 5 to 10 and for example equal to 6;
- p and m are independently equal to 0 or 1, in particular equal to 0; and
- when p and/or m is(are) equal to 1, R³ and R⁴ independently represent a halogen atom, for example selected from an iodine atom, a chlorine atom and a bromine atom;
and more particularly the organometallic complex comprises at least, notably consists of, the compound of formula **(II'):**

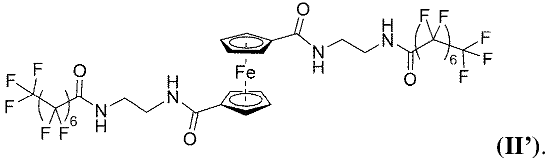

6. Nanovector according to any of the preceding claims, which core further comprises at least one near infrared-responsive photosensitizer suitable to produce oxidizing species when exposed to near infrared light and being functionalized by at least one hydrophobic moiety, optionally at least one hydrophobic perfluorinated moiety,
wherein the near infrared-responsive photosensitizer comprises in particular at least one group chosen from phenothiazines, porphyrins, chlorins, bacteriochlorins, cyanines, phtalocyanines, pyropheophorbide-a or derivatives thereof, more particularly a pyropheophorbide-a group or a derivative thereof, even more particularly is a compound of formula **(III):** 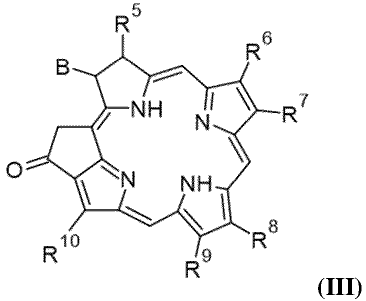
wherein:
- R⁵, R⁶, R⁷, R⁸, R⁹ and R¹⁰ independently represent a hydrogen atom, a (C₁-C₆)alkyl group or a (C₂-C₆)alkenyl group; and
- B represents a hydrophobic moiety, in particular comprising a substituted or unsubstituted, saturated or unsaturated, aliphatic C₄-C₃₆ chain and/or a hydrophobic perfluorinated moiety,
or a mixture of compounds of formula (III);
in particular wherein:
- R⁵, R⁶, R⁸, R⁹ and R¹⁰ independently represent a (C₁-C₄)alkyl group;
- R⁷ represents a (C₂-C₄)alkenyl group;
- B comprises a hydrophobic perfluorinated moiety, in particular B represents a -(CH₂)ᵣ-X-(CH₂)ₛ-Y-(CF₂)ₜ-CF₃ group, wherein
- r and s are independently equal to 0, 1, 2, 3 or 4, in particular equal to 1, 2 or 3,
- t is equal to 1 to 25, in particular equal to 3 to 15, more particularly equal to 5 to 10 and even more particularly equal to 6, and
- X and Y independently represent a single bond, a -O- group, a -NH- group, a - C(=O)- group, a -C(=O)-O- group, a -O-C(=O)- group, a -NH-C(=O)- group, or a
- C(=O)-NH- group, in particular a -C(=O)-O- group, a -O-C(=O)- group, a -NH-C(=O)- group, or a -C(=O)-NH- group,
more particularly said near infrared-responsive photosensitizer comprises at least, notably consists of, the compound of formula (III'):

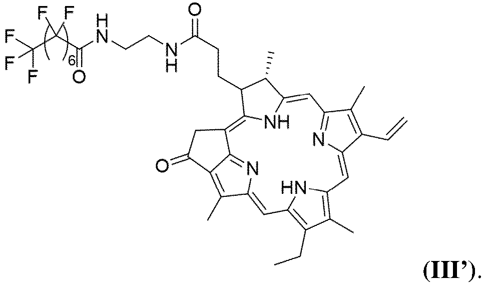

7. Nanovector according to any of the preceding claims, which core further comprises at least one probe for magnetic resonance imaging, in particular at least one fluorinated probe for ¹⁹F magnetic resonance imaging, more particularly said fluorinated probe comprises at least one perfluorinated moiety, even more particularly said fluorinated probe comprises a compound chosen from hexafluorobenzene (HFB), perfluorodecalin (PFDC), perfluorononane (PFN), perfluoroctyl bromide (PFOB), perfluoro-15-crown-5-ether (PFCE), perfluoropolyethers (PFPEs), N-(2,2,2-trifluorethyl)acrylate (TFEA), N-(2,2,2-trifluorethyl)methacrylate (TFEMA), 2,3,4,5,6-pentafluorostyrene (PFS), octafluoropentyl methacrylate (OFPMA), N-(2,2-difluorethyl)acrylamide (PDFEA), 1,3-bis[[1,1,1,3,3,3-hexafluoro-2-(trifluoromethyl)propan-2-yl]oxy]-2,2-bis[[1,1,1,3,3,3-hexafluoro-2-(trifluoromethyl)propan-2-yl]oxymethyl]propane (PERFECTA) and mixtures thereof, and more preferably the fluorinated probe comprises at least, notably consists of, the compound PERFECTA of formula (IV):

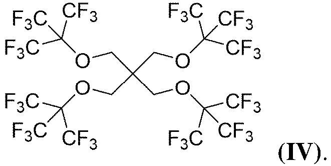

8. Composition, comprising water and a plurality of nanovectors as defined in any of the preceding claims, the amphiphilic molecules being in particular present at a concentration superior or equal to the critical micelle concentration of said amphiphilic molecules, more particularly the composition comprising at least 0,1 g/L of amphiphilic molecules, even more particularly from 5 g/L to 50 g/L of amphiphilic molecules.

9. A pharmaceutical or diagnostic composition, comprising a plurality of nanovectors as defined in any of claims 1 to 7 and at least one pharmaceutically acceptable excipient.

10. A method for preparing nanovectors, in particular as defined in any of claims 1 to 7, comprising at least the steps of:
a) providing an aqueous composition comprising:
- a plurality of amphiphilic molecules, said amphiphilic molecules comprising at least one hydrophobic moiety linked to at least one hydrophilic moiety, in particular as defined in claim 3 or 4, more particularly as defined in claim 4;
- at least one organometallic complex having a paramagnetic property that can be activated by oxidation, said organometallic complex being functionalized by at least one hydrophobic moiety, in particular as defined in claim 5;
- optionally at least one near infrared-responsive photosensitizer suitable to produce oxidizing species when exposed to near infrared light and being functionalized by at least one hydrophobic moiety, in particular as defined in claim 6; and
- optionally at least one probe for magnetic resonance imaging, in particular at least one fluorinated probe for ¹⁹F magnetic resonance imaging, more particularly as defined in claim 7; and
b) mixing, in particular ultra-sonicating, the aqueous composition of step a); thereby preparing the nanovectors.

11. A nanovector according to anyone of claims 1 to 7, or a composition according to claim 8, or a pharmaceutical or diagnostic composition according to claim 9, for use in *in vivo* diagnostics.

12. A nanovector according to anyone of claim 1 to 7, in particular according to claim 7, or a composition according to claim 8, or a pharmaceutical or diagnostic composition according to claim 9, for use in a method of magnetic resonance imaging, in particular ¹⁹F magnetic resonance imaging.

13. A method of magnetic resonance imaging also called MRI, in particular ¹H-MRI, ¹⁹F-MRI, ¹³C-MRI, ²H-MRI or ³¹P-MRI, more particularly ¹⁹F-MRI, comprising at least the steps of:
i) exposing to light, in particular blue light and/or near infrared light, more particularly at a wavelength comprised between 300 nm and 1200 nm, even more particularly between 400 nm and 800 nm, for example between 400 nm and 700 nm, zone(s) of a patient previously administered with nanovector(s) according to anyone of claims 1 to 7, in particular with a composition according to claim 8 or a pharmaceutical or diagnostic composition according to claim 9, said zone(s) being not to be imaged by MRI; and
ii) imaging zone(s) of interest of said patient by MRI, in particular ¹H-MRI, ¹⁹F-MRI, ¹³C-MRI, ²H-MRI or ³¹P-MRI, more particularly ¹⁹F-MRI, the zone(s) of steps i) and ii) being distinct,
step ii) being consecutive or simultaneous with step i).

14. A method of MRI according to the preceding claim, wherein said light at step i) is near infrared light, in particular at a wavelength comprised between 600 nm and 1200 nm, more particularly between 600 nm and 800 nm, even more particularly between 620 nm and 700 nm, and nanovector(s) are according to claim 6 or 7.

15. A method of MRI according to claim 13 or 14, wherein the core of the nanovector(s) further comprises at least one fluorinated probe for ¹⁹F magnetic resonance imaging, said nanovector(s) being in particular according to claims 7, and MRI is ¹⁹F-MRI.

16. A kit for magnetic resonance imaging, in particular for ¹H-MRI, ¹⁹F-MRI, ¹³C-MRI, ²H-MRI or ³¹P-MRI, more particularly for ¹⁹F-MRI, comprising:
- a plurality of amphiphilic molecules, said amphiphilic molecules comprising at least one hydrophobic moiety linked to at least one hydrophilic moiety, in particular as defined in claim 3 or 4, more particularly as defined in claim 4;
- at least one organometallic complex having a paramagnetic property that can be activated by oxidation, said organometallic complex being functionalized by at least one hydrophobic moiety, in particular as defined in claim 5;
- optionally at least one near infrared-responsive photosensitizer suitable to produce oxidizing species when exposed to near infrared light and being functionalized by at least one hydrophobic moiety, in particular as defined in claim 6; and
- optionally at least one probe for magnetic resonance imaging, in particular at least one fluorinated probe for ¹⁹F magnetic resonance imaging, more particularly as defined in claim 7.
